# EUROPEAN PATENT APPLICATION

(11) **EP 4 393 470 A1**
(43) Date of publication of application: **03.07.2024**
(21) Application number: 22217391.6
(22) Date of filing: 30.12.2022
(51) Int. Cl.: A61K 8/44, A61K 36/886, A61K 31/205, A61K 8/9794, A61Q 5/02, A61Q 19/00, A61P 17/00, A61P 17/04, A61P 17/06, A61P 17/08, A61P 17/12, A61P 17/16

(54) **PLANT-MINERAL COMPOSITION BASED ON ALOE BARBADENSIS EXTRACT AND TRIMETHYLGLYCINE FOR PH-DEPENDENT REGULATION OF TYPE 3 AQUAPORINS AND TRANSEPIDERMAL WATER FLOW IN DEEP EPIDERMAL LAYERS**

(71) Applicant: SkyLab AG, 1066 Epalinges (CH)
(72) Inventor: Varava, Andrey Vyacheslavovich, 125171 Moscow (RU); Filatov, Viktor Andreevich, 119415 Moscow (RU); Belous, Elena Yur'evna, 121309 Moscow (RU)
(74) Representative: Glawe, Delfs, Moll

(57) **Abstract**

The invention relates to a composition comprising a complex of plant extract from *Aloe Barbadensis* and N-trialkyl glycine derivative in a specific raw material ratio and the raw material ratio equivalent to active ingredients and the use of said composition as a component of a body care product or as a pharmaceutical preparation and/or for treatment of a subject suffering from a skin wound or disease.

## Description

### ART

The invention is in the class of cosmetology and is a biocomplex for skin and scalp care, particularly for pH-dependent regulation of Type 3 aquaporins in all epidermal layers and for deep distribution of transepidermal water flow in skin cells of the body and the scalp without damaging viscoelastic-plastic properties of epidermis. The invention is in the class of biocomplex and its application in composition of cosmetic products; the application is composed of components A and B, where:
(A) Plant extraction from *Aloe Barbadensis,* in particular, *Aloe Barbadensis* leaf extract;
(B) N-trialkylated glycine derivative, in particular, trimethylglycine, where mass ratio equivalent to active ingredients makes (0.0001-1.00) : (0.1-10) weight %, correspondingly.

The biocomplex is designed to be introduced to the composition of cosmetic products for skin and scalp care, the use of which allows increasing the quantity of Type 3 aquaporins (AQP 3) in keratinocytes and regulate transepidermal water flow in all epidermal layers. The complex is of natural origin; biodegradable; has a safe effect on sensitive skin and can be used for the production of such cosmetic products as liquid shower gels, scalp and hair shampoos, hair balms and conditioners, anti-dandruff, seborrheic and xerosis shampoos; liquid hand washes, face washes, face creams, hand creams, skin serums, solid products for skin, hand and hair care. The use of the complex helps reducing transepidermal water loss, increasing the amount of Type 3 aquaporins (AQP 3) in all epidermal layers for long-term prevention of dry skin, caused by specific impact of various environmental factors and by the use of unsuitable cosmetic ingredients in the product composition, as well as deep cell humidifying and regulating pH gradient of hydrolipidic mantle.

### BACKGROUND OF THE INVENTION

Development of cosmetic products with properties, improved for customers, remains one of the promising areas in the category of skin, face and hair care products. Growing stress and concern about provision of ingredient and product safety due to COVID-19 caused customers to select products for dry and sensitive skin. After COVID-19 outbreak, the output of sensitive skin care products continues to grow all over the world; these products include well-known ingredients and new combination of natural ingredients, used to create safe, highly efficient and multifunctional products. By 2023, the market value of skin care in the Russian Federation will achieve 5 bln. $; the projected growth will make 17.0% CAGR for 5 years. [Mintel Market Sizes, Body care, Facial care - multiple sizes, 2018]. The "hydrating/moisturizing" care segment makes over 14% of the sales pattern for skin and face care in the Russian Federation, whereas in Germany, France, China, India, the USA and Great Britain it makes 42%, 35%, 24%, 21%, 15% and 12% correspondingly, which proves the timeliness of development of the products with scientifically proved features of skin moisture content regulation.

According to Mintel analytical database and online poll, over 59% consumers in China and 26% of consumers in Germany aged 18-49 selects only dry skin care [KuRunData/Mintel Lightspeed/Mintel]. Over 42% consumers form Germany prefer multifunctional skin moisturizing products [Lightspeed/Mintel; Cipher/Mintel]. Due to COVID-19 pandemic, dry skin and eczema increase after using deep cleaning and disinfecting products; this is why over 20% consumers in Great Britain aged 16-24 select only moisturizing and softening skin care [Lightspeed/Mintel; Cipher/Mintel; The Journal of the American Academy of Dermatology].

Consumers from various parts of the world look for natural and efficient skin and face care products, which would contain innovative moisturizing components. Thus, the survey of 3000 consumers in China aged 18-49 demonstrated that over 46% of them are looking for innovative moisturizing products with scientifically proved action and sufficient rationale for the advantages [Mintel, A year of innovation in body, hand and footcare, 25.05.2022]. Consumers from Pacific Asia are looking for skin and face care with innovative combination which would be also effective in rinse-off products [https://clients.mintel.com/report/a-year-of-innovation-in-body-hand-and-footcare-2022?fromSearch=%3Ffreetext%3Dmoisture&resultPosition=19]. According to the analytical results by, the launch of cosmetic products labelled as "moisturizing" made 71% in average on the territory of Asia Pacific and Australasia Countries, and the number of products labelled as "for sensitive skin" increased by 10% for the past 4 years. This trend is also observed in Europe, Middle East, and Africa and in North America.

However, in spite of the demand for effective products, consumers keep focusing on dermatological comfort of hand skin after using cosmetic products. Every day, a person uses large quantity of hygienic products and cleansing products for face and body. To protect oneself against the development of dermatological diseases and to prevent the occurrence of xerosis, it is necessary to pay attention to selecting products with innovative moisturizing combinations, as it is dermatological comfort of body and face skin that is the key to health and correspondingly to the high quality of life. Due to frequent contact of skin with synthetic components in products with high content of natural ingredients with scientifically proved effects remains the most reliable method of maintaining healthy skin and, correspondingly, healthy skin look. According to the specialists, skin health is one of the fundamentals of human health. Thus, 2% sodium lauryl sulfate, known as SLS, in the composition of body cleansers, is capable to cause loss of transepidermal water by 68.9 g/m2/h in comparison with the normal level in 12 hours after the contact with the product [Loffler, H., & Happle, R. (2003). Profile of irritant patch testing with detergents: sodium lauryl sulfate, sodium laureth sulfate and alkyl polyglucoside. Contact Dermatitis, 48(1), 26-32]. As the content of sodium lauryl sulfate in cleansers can reach 10% due to high cleansing activity, it may negatively impact the condition of body and scalp skin. Petrochemical surfactants also cause keratin denaturation due to decomposition of sulphide bonds and generation of sulfhydryl groups-SH on human cells and elution of epidermal barrier [Prottey C, Ferguson T. Factors which determine the skin irritation potential of soaps and detergents. J Soc Cosmet Chem. 1975;26: 29-46.]. It was made clear that aggressive anionic surfactants, sodium decyl sulfate (SDS), sodium myristyl sulfate (SMS), sodium tridecyl sulfate (STS) cause elution of water-soluble protein from epidermic by 166.1%, 163.9% and 198.5%, correspondingly [Loffler, H., & Happle, R. (2003). Profile of irritant patch testing with detergents: sodium lauryl sulfate, sodium laureth sulfate and alkyl polyglucoside. Contact Dermatitis, 48(1), 26-32], which is manifested in dry, tight skin and erythema. Thus, development of cosmetic products with moisturizing components to increase moisture content in skin cells and to regulate transepidermal water flow in skin is a priority area in caring about dermatological comfort of face, body and hair.

### STATE OF THE ART

Skin is the largest body organ, taking 1/6 of the whole body mass, which contacts the environment and perceives signals. It is a complicated organ, performing an important barrier function, on the surface of which microbial communities symbiotically intercorrelate with the host's tissue through immunity mechanisms. A human skin is continuously exposed to various endogenic and exogenic factors, which may result in the development of inflammations: UV and other radiation, contamination and heavy metals, high and low temperatures, microelemental composition of air, humidity, as well as stress and vitamin deficiency. In recent studies, scientists try to combine information about physical, immunological and physiological parameters of this organ into a single concept about barrier functions [187].

From the physiological point of view, skin is a complex physical and chemical structure with a set of various features (mechanical, electromagnetic, optical, chemical, biochemical), being in open and constant interaction with the environment. When normal skin is described by scientific society, they mean a whole complex of parameters, which also include an integral skin barrier, normal sebum regulation and microbiota balance on epidermal surface. Epidermis is a five-layered surface layer of skin, the cells of which are constantly diving and moving to the surface, forming corneal layer of skin. It is the corneal layer which defines the barrier function of skin, the condition of microbiota, maintenance of pH balance and moisture condition, as damage to epidermis integrity results in excessive transepidermal water loss (TEWL).

The first protective layer challenged by the environment is an acid skin barrier, named hydrolipidic mantle, or Marcionini's mantle. It is composed of glands' excretory products, epidermic cells and microorganisms: free fatty acids of skin sebum, lipids, cholesterol, squalene, urocaninic acid, pyrrolidine carboxylic acid and other components of natural moisturizing factors; a mixture of sweat, organic acids (lactic, citric acid), antimicrobial peptides, lipid preoxygenation products. Thanks to these biochemical products, skin surface has weak acid pH value, from 4.5 to 5.9, depending on regional specific features. This value plays the key role in maintaining skin moisture, protecting skin from mechanical and chemical damage and from pathogenic microorganisms, as well as in maintaining viscoelastic-plastic properties of epidermis. Skin sebum and sweat also have pH about 5.5 and create hyperosmotic environment, unfavorable to excessive microbial growth. It is worth mentioning that body skin and scalp are different in favorable pH: the most favorable pH for scalp skin is 4.75-5.04, whereas the one for various body areas is 5.06-5.90 [Dréno B., Araviiskaia E., Berardesca E. et al. Microbiome in healthy skin, update for dermatologists // Journal of the European Academy of Dermatology and Venereology, 2016].

Maintaining pH balance of skin is impacted by various factors, namely age, sex, genetic traits of enzyme system functioning, ethnical differences, indoor humidity, composition of skin flora and indoor microbiome, excretion of perspiratory and oil glands, as well as applied cosmetic products and medicinal drugs, circadian rhythm, nutritional habits and wearing protective gear.

Physiological pH of the corneal layers is determined by balance between cell and metabolic processes, generating or consuming H+ protons, while the major role is played by lipid metabolism on epidermal surface. The significance of epidermic pH is the necessary determinant for lipid synthesis by the epidermal corneal layer, as it determines activity of enzymes, catalyzing transformation of lipid predecessors into ceramides, free fatty acids or cholesterol. Also, pH condition has a paramount importance for molecular structure and physical and chemical characteristics of membranes, e.g. membrane fluidity and membrane permeability. Researchers in dermatology and trichology determined sigmoidal relationship of pH gradient on the corneal layer surface and enzyme activity, defining the current condition of epidermal layer. For pH-dependent enzymes, in particular, β-glucocerebrosidase and acid sphingomyelinase, the optimal pH value is in the range of 5.6 and 4.5, correspondingly. It was found out that β-glucocerebrosidase activity is 10 times less when pH is 7.4, than when pH is 5.6; in this case excessive activity of serine protease, in particular, kallikrein 5, 7 and alkaline ceramidase.

Modifications of acid-alkaline balance in tenth and hundredth units of pH, from weak acid to neutral zone, not only inhibit regeneration metabolic pathways, but also activate enzymes, providing accelerated decomposition of the corneal layer and peeling, namely serine protease KLK5 and KLK7, alkaline ceramidase. Alkaline enzymes provide peeling and excessive degradation of lipid bilayer, which is evidence by increased TEWL parameter, itching, increased sensitivity and painful sensation, sculp erythema. This important biochemical aspect allows describing the occurrence of visible horny scales in dermatitis, which a person tries to rinse with various cleansers.

Corneal layer acidity is relatively constant thanks to the buffer capacity of skin itself. Skin buffer system is not widely recognized by scientists; however, it has a critical importance in correct differentiation of corneocytes and preservation of lipid lamellas. The main components with buffer capacity include weak organic acids, fatty acids, urocaninic and lactic acids, amino acids, peptides, keratin and antimicrobial peptides. In adult skin, buffer capacity is rather high, which allows adults withstanding excessive cleaning with alkaline products; however, infants, elderly people and patients with dermatological diseases have increased skin sensitivity even to surface-active agents (SAAs) in the composition of shampoos. This is why pH and buffer capacities of products for external use are especially important. It is necessary to consider that skin pH after product application is the result of joining pH of buffer capacity of both the product and the target corneal layer. In perspective pharmaceutical developments it is worth paying attention to high buffer capacity of the medicinal drug to maintain the required pH of the corneal layer for several hours after application. The use of cosmetic products with weak acid pH 4.5-5.7 and acid-based buffer system will allow maintaining skin pH, shift the balance to normal peeling and integral lipid layer of the scalp, as well as to provide favorable conditions for the development of residential skin microbiota.

Skin performs its functions only in case its barrier layers is not damaged and sufficient transepidermal water flow is provided. Water is vitally important for natural functioning and healthy skin look, as skin is the largest external body organ with three separate layers: epidermis, dermis and hypodermis. Skin moisturizing is the result of interaction of the three basic mechanisms: the corneal layer and its barrier role in relation to water loss, natural moisturizing factor (NMF), including several hygroscopic molecules to maintain corneocytes hydration, and water transportation channels. The epidermal corneal layer acts as the primary protective barrier against physical, chemical and ecological impact. In particular, intracellular lipids and natural moisturizing factor help maintaining skin moisture, preventing TEWL. NMF is composed of various amino acids and other hygroscopic molecules, resulting from proteolysis of filaggrin and other components of the corneal layer. Damage to the epidermal corneal layers due to insufficient NMF, water and severe environmental conditions (UVA or UVB) worsens dry skin and related dermatological disorders, such as dermatitis, itching, psoriasis, lesions and ageing. However, even if the barrier layer is preserved and NMF is sufficient, water must be provided and distributed between cells for normal functioning.

2003 Nobel Prize for Chemistry was given for research, directly related to biology, specifically to life support of a living cell, an elementary unit of biological entities. The discoveries made by two American scientists, Peter Agre and Roderick MacKinnon, allowed thorough detailing in how salt and water metabolism of a cell with the environment is performed. Every living cell is surrounded with a membrane, consisting of a double lipid layer with protein inclusions. An extraordinary feature of such membrane is its selective permeability. It permits everything which is necessary for life support, including water, while effectively protecting the cell from the hostile environment. As far back as in mid-19^{th} century scientists proposed that a cell envelope has pores for water penetration, however an opinion was prevailing for a long time that water just diffuses through cell membrane.

In late 1950s it was found out that cell membranes have special channels, which permit water, but not ions. With this, cellular "water supply" has a remarkable channel capacity, up to 1 billion water molecules per second. It would be reasonable to assume that, as in case with other substances, as sugars and amino acids, transportation of water through a cell membrane occurs with protein. Discovery of aquaporins, which are proteins generating water pores, occurred by way of chance. In 1987, Peter Agre was studying erythrocyte antigen proteins and discovered membrane protein with an unknown function. It turned out that the same protein is abundant in renal tubules capable of pumping huge amounts of water. This made the scientist think that the discovered protein is related to water transportation through a cell membrane. Peter Agre and his colleagues were able to determine the amino acid sequence of the protein and then cloned a DNA section coding aquaporin synthesis. The scientists performed several experiments which undoubtedly proved the key role of this protein in water transportation. For instance, if we "make" a cell to produce large amounts of aquaporin, it starts to intensively absorb water, distributes it between other cells and regulates salt and water metabolism.

The space structure of aquaporin resembles a cylinder channel, in which water molecules move. Only water, but not ions, comes through it. Amino acids in protein are located in the manner that the polarity of the generated electrostatic field is switched to reversed polarity in the center of a molecule. This is why water molecules, reaching the middle of the channel, turn around so that their dipolar moments in the upper and the lower parts of the channel are in the opposite directions. Such re-orientation prevents ion penetration through the channel. Aquaporin does not pass even H3O⁺hydronium ions (i.e., hydrated protons), the concentration of which affects acidity.

The discovered protein is the first, but not the only one from aquaporin family. By now, about 200 varieties of water channel proteins in plants and animals, including 11 varieties in humans, have been known. Thanks to aquaporins, cells not only regulate its volume and inner pressure but also perform such important functions as water absorption in animals' kidneys and plants' roots. In case of skin, such proteins are also present, providing gene deep natural transepidermal water flow. Type 3 aquaporins (AQP 3) are the only proteins in skin cells, supplying water to cells for skin moisturizing. When an amount of aquaporins in skin is small, or moisture is insufficient, or skin barrier is damages (peeling, dermatitis, excessive mechanical skin cleaning with a scrub or a brush, synthetic SAAs etc.), skin becomes dry, sensitive, irritant and requires moisturizing. A discovery by 2003 Nobel Prize winners helped to understand the reason for dry skin. Aquaporin-3 operates in cells and protects skin from dehydration, due to which is it of scientific interest to search for original combinations designed to increase the amount of aquaporins.

Normal expression of AQP3 is necessary for normal transepidermal water flow in cells, TEWL decrease, skin barrier restoration, wound healing and skin elasticity. Various external and internal factors influence the aquaporin activity in epidermal cells: pH [Tournaire-Roux C., Sutka M., Javot H. et al. Cytosolic pH regulates root water transport during anoxic stress through gating of aquaporins // Nature, 2003;425:393-397], heavy metals [Zelenina M., Tritto S., Bondar A.A. et al. Copper inhibits the water and glycerol permeability of aquaporin-3 // J. Biol. Chem., 2004;279:51939-51943.], bivalent metal cations [Nemeth-Cahalan K.L., Hall J.E. Ph and calcium regulate the water permeability of aquaporin 0 // J. Biol. Chem, 2000;275:6777-6782.], cell membrane tension [Ozu M., Dorr R.A., Gutierrez F. et al. Human aqp1 is a constitutively open channel that closes by a membrane-tension-mediated mechanism // Biophys. J, 2013;104:85-95]. It was found out that, activity of Type 3 aquaporins in skin cells is pH-dependent, which emphasizes the necessity of pH regulation to maintain the activity of Type 3 aquaporins in skin cells and to regulat4e the integrity of the skin barrier layer. At pH <4.0, AQP3 activity decreases completely due to changes in protein structure and low permeability for water molecules. The use of pH 5.0 and more provides significant increase of AQP3 activity even in the deepest epidermal layers [Mósca A., de Almeida A., Wragg D. et al. Molecular Basis of Aquaporin-7 Permeability Regulation by pH. // Cells, 2018, 7(11), 207-227]. The optimal value of acidity is pH 5.0-7.0 to maintain basic AQP3 activity in basal and superbasal epidermal layers to maintain significant amount of water and transepidermal water flow.

Under the environmental conditions, both the integrity of skin barrier and the ability to maintain and pass water in epidermis are damaged. It is found out that skin aging is related to decrease of moisture in skin, increased TEWL parameter and decreased amount of Type 3 aquaporins in epidermal cells [Ikarashi N., Kon R., Kaneko M. et al. Relationship between Aging-Related Skin Dryness and Aquaporins // International Journal of Molecular Sciences, 2017]. Moreover, tight contacts between epidermal cells are decreased, which means that skin is not a tight barrier and loses excessive water amount [Sylvie Verdier-Sévrain, Frédéric Bonté Skin hydration: a review on its molecular mechanisms // Journal of Cosmetic Dermatology, 2007, 6(2), 75-82].

The connection of AQP3 amount with various dermatological diseases, such as atopic dermatitis [Boury-Jamot M., Sougrat R., Tailhardat M. et al. Expression and function of aquaporins in human skin: Is aquaporin-3 just a glycerol transporter? // Biochim Biophys Acta, 2006;1758:1034-1042], psoriasis [Qin H, Zheng X, Zhong X. et al. Aquaporin-3 in keratinocytes and skin: Its role and interaction with phospholipase D2 // Arch Biochem Biophys, 2011;508:138-143], vitiligo [Kim N-H., Lee A-Y. Reduced aquaporin3 expression and survival of keratinocytes in the depigmented epidermis of vitiligo // J Invest Dermatol 2010;130:2231-2239] and chronic skin irritation [Ikarashi N., Ogiue N., Toyoda E. et al. Gypsum fibrosum and its major component CaSO4 increase cutaneous aquaporin-3 expression levels. J Ethnopharmacol 2012;139(2):409-413] is specified. In the development of psoriasis, the decrease of AQP3 amount due to excessive immunity reaction is specified [Choudhary V., Olala Lawrence O., Kagha K. Et al. Regulation of the glycerol transporter, aquaporin-3, by histone deacetylase-3 and p53 in keratinocytes // Journal of Investigative Dermatology, 2017]. In case skin is not sufficiently moisturized, increased expression of Type 3 aquaporins is observed; however, it is short-term and adaptive for a short period of time [Brandner J.M. Pores in the epidermis: aquaporins and tight junctions // Int J Cosmetic Sci, 2007:29:413-422]. Scientists detected that increased AQP3 expression in skin cells accelerates wound healing due to supply of water, glycerin and keratinocyte migration [Sebastian R., Chau E., Fillmore P. et al. Epidermal aquaporin-3 is increased in the cutaneous burn wound // Burns, 2015, 41(4), 843-847]. Stimulation of AQP3 expression can be a potential mechanism to prevent and treat these conditions. Due to the important role of AQP3 in skin and the necessity of regulation of AQP3 amount in epidermal skin, the search for innovative combination for long-term skin moisturizing, keratinocytes proliferation and skin barrier function maintenance remains urgent [Hara M., Ma T., Verkman AS. Selectively reduced glycerol in skin of aquaporin-3 deficient mice may account for impaired skin hydration, elasticity and barrier recovery // J Biol Chem, 2002;277:46616-46621].

Search for effective components and their combinations as formulation for moisturizing and TEWL decrease is a routine task for companies, involved in the production of cosmetic products for face, body and scalp care. The main components of modern products for skin moisturizing are occlusal agents, moisture retaining components, film coating agents, emollients, performing limited functions. The most perspective approach is the use of components, regulating gene deep mechanisms of natural moisturizing and not damaging skin barrier function. In particular, such functional additives include *Aloe Barbadensis* leaf extract and N-trialkylated glycine derivative, in particular, trimethylglycine or betaine.

Extracts of various plants are irreplaceable components in hand care products due to their antibacterial, antioxidating, moisturizing and protective features. Thanks to benign impact on hand skin, high efficiency of the whole complex of biologically active substances (BAS) and low frequency of adverse effects after use, if compared with the use of medicinal drugs, plant-based extracts make the foundation of modern skin care products, in particular, for body skin, face and sculp.

*Aloe Barbadensis Leaf Extract* (CAS 85507-69-3/94349-62-9, EC 287-390-8/305-181-2) is a water-soluble extract made of aloe vera leaves, containing various biologically active substances from the group of carbohydrates, lipids, fatty acids and proteins. *Aloe Barbadensis* leaf extract is known for their skin soothing and local lesion mitigating properties. However, phytochemical properties, detected in *Aloe barbadensis,* are not sufficiently studied in terms of their dermatological advantages [S.Kumar Kar, T. Kanti Bera. Phytochemical constituents of aloe vera and their multifunctional properties: a comprehensive review // Int J Pharm Sci Res, 2018; 9(4): 1416-23]. By inhibiting inflammatory mediators, B2 thromboxane and F2 prostaglandin, with bradikynase, *Aloe barbadensis* extract helps to reduce excessive inflammation in local administration on hand skin [Keisuke Fujita, Ryoji Teradaira, & Toshiharu Nagatsu. (1976). Bradykininase activity of aloe extract. Biochemical Pharmacology, 25(2), 205]. Moreover, *Aloe Barbadensis* leaf extract participates in protecting skin from free radicals. Aloin in the extract composition can neutralize up to 30% UV radiation when applied on skin surface. *Aloe Barbadensis* is also enriched with vitamins C and E, which are also antioxidants, and which protect skin from harmful effect of ultraviolet, environmental contamination and chemical irritants [Jia, Y., & Jia, J. Ultraviolet Light Absorption of an Ophthalmic Formulation with Aloe Extracts // Natural Product Communications, 2009, 4(9)].

In the randomized double-blind placebo-controlled study, subjects were studied to estimate effects of using phytosterols from *Aloe barbadensis* on such skin parameters, as elasticity, moisture and collagen content in the extracellular matrix. After 12 weeks of the study, a statistically significant improvement was specified (p<0.05) in skin elasticity by means of increasing collagen synthesis, as phytosterols have estrogen-like action and provides activation of transcriptional factors, regulating the activity of dermal procollagen I genes [Tanaka M., Yamamoto Y., Misawa E. et al. Effects of aloe sterol supplementation on skin elasticity, hydration, and collagen score: A 12-week double-blind, randomized, controlled trial // Skin Pharmacol. Physiol., 2016, 29, 309-317]. In the similar study with participation of 48 healthy men, a significant improvement of skin elasticity was detected [Tanaka M., Yamamoto Y., Misawa E. et al. Aloe sterol supplementation improves skin elasticity in Japanese men with sunlight-exposed skin: A 12-week double-blind, randomized controlled trial // Clin. Cosmet. Investig. Dermatol., 2016, 9, 435-442]. During in vitro studies of alcoholic *Aloe barbadensis* lead extract, rich in hexadecanoic acid (22,22 %), sitosterol (2,89 %) and stigmasterol (2,10 %), antioxidant activity was detected with DPPH text - 5.2 mg/ml. FRAP analysis of *Aloe barbadensis* leaf extract showed high neutralizing activity to hydroxyl radical OH. - IC50 made 1.17 µg/ml, and minimal concentration of the extract to prevent lipid peroxygenation made 4.18 µg/ml. The extract also showed antimicrobial effect to *S. greseus* and *C*. *albicans* on skin [Bawankar R., Deepti V. C., Singh P. et al. Evaluation of Bioactive Potential of an Aloe vera Sterol Extract // Phytotherapy Research, 2012, 27(6), 864-868]. Thus, phytosterols from *Aloe barbadensis* thanks to estrogen-like action on ERBα and β receptors of fibroblasts of hand dermis, provide collagen synthesis, skin firmness and skin look improvement, which is an additional achievement of using *Aloe barbadensis* in composition of cosmetic products for skin care.

Vitamin C, the large amount of which is available in *Aloe barbadensis* leaves also plays its role in collagen synthesis, thanks to which skin surface remains strong and firm. Moreover, Aloe *barbadensis* includes β-carotin, which is a precursor to vitamin A, which demonstrates expressive antioxidating activity [S.Kumar Kar, T. Kanti Bera. Phytochemical constituents of aloe vera and their multifunctional properties: a comprehensive review // Int J Pharm Sci Res, 2018; 9(4): 1416-23]. *Aloe barbadensis* leaf juice contains superoxide dismutase (SOD) and glutathione peroxidase (GxP), which are accepted strong antioxidants and cell anti-aging agents, and proline, an amino acid, which is important for skin youth and plays an important role in providing retention capacity and elasticity of epithelial tissues [Surjushe A, Vasani R and Saple DG. Aloe vera: A short review // Ind. J. Dermatol., 2008; 53: 163-166]. Thus, *Aloe Barbadensis* leaf extract participates in maintaining young skin in external use thanks to the complex of biologically active substances.

*Aloe barbadensis* contains glycosaminoglycans (GAG) from the sugar class, participating in water and skin bonds. GAG, being a human skin component, acts as support for collagen and elastin in dermis. Collagen, elastin and GAG form a larger part of extracellular matrix. In combination with some proteins, glycosaminoglycans are able to bind water and other cell elements, thus making an integral monolithic matrix, which supports skin cells together and assists in performing skin barrier function. Preservation of this intercellular layer does not let bacteria penetrate into skin, does not emit water and maintains evenness and smoothness of skin surface.

An antiseptic property of *Aloe barbadensis* is explained by the presence of various antiseptic agents, particularly lupeol, salicylic acid, urea nitrogen, cinnamylic acid, phenol and sulphur. These compounds produce an inhibitive action on fungi, bacteria and viruses [Boudreau M.D., Beland F.A. An evaluation of the biological and toxicological properties of Aloe barbadensis (Miller), Aloe vera // J. Environ. Sci. Health., 2006; 24: 103-154]. Acemannan is the main carbohydrate fraction, received from *Aloe barbadensis* leaves, which helps cells to be more resistant to viruses and pathogenic bacteria, produces anti-inflammatory effect, provides faster wound healing, decreases allergic skin reactions and has anti-aging effect [Sierra-Garcia G. D., Castro-Ríos R., González-Horta A. et al. Acemannan, an Extracted Polysaccharide from Aloe vera: A Literature Review // Natural Product Communications, 2014, 9(8)].

Thus, *Aloe barbadensis* has a whole spectrum of favorable effects for human skin, starting from antibacterial and anti-inflammatory function and finishing in participation of its components in promoting young skin. However, nothing is known about long-term of aloe vera effect on pH-sensitive regulation of the amount of Type 3 aquaporins in epidermal cells and increase of transepidermal water flow in upper skin layers, which emphasizes the necessity of developing an innovative complex for regulation of long-term moisturizing mechanism for skin's own cell renew and maintenance of skin barrier function.

N-trialkylated glycine derivative, trimethylglycine or betaine (CAS 107-43-7, EC 203-490-6), is a water-soluble substance, derived from sugar beet roots [Gottschalck T.E. and Breslawec H.P. International Cosmetic Ingredient Dictionary and Handbook. 14 ed. Washington, DC: Personal Care Products Council, 2012]. It is a 100% natural osmolyte, which is naturally present in all living cells, especially in beet root cells. Osmolytes perform the two basic functions: control and regulate osmotic processes to keep intracellular conditions for optimal metabolic activity and protect macromolecular structures from osmotic stress.

Trimethylglycine plays an important role not only in protection of skin from osmotic stress but in assistance in protein folding and native protein structure stabilization. Thanks to water binding properties, trimethylglycine is able to make upper skin layers, particularly epidermic corneal layer, soft and elastic. Addition of trimethylglycine increases synthesis of tight contact proteins in skin and further restoration of skin barrier function. Addition of 35 mM trimethylglycine increases expression of transporter of BGT osmolarity, increasing water retaining features of skin cells themselves (p<0.001), as well as expression of claudin-1 (p <0.001), claudin-4 (p=0.0012) and occludin (p<0.001) protein [C. El-Chami, A.R. Foster, C. Johnson et al. Organic osmolytes increase expression of specific tight junction proteins in skin and alter barrier function in keratinocytes // British Journal of Dermatology, 2020].

Keratinocytes use osmolytes to maintain their amount and protection from environment, such as UV-radiation and thermal stress. In dry skin, keratinocytes are in the conditions of hyperosmotic stress. The direct sequence will be water outflow and further cell shortage. Keratinocytes respond by increasing production of osmolytes' carriers on their cell membranes, which results in increased consumption of osmolytes. This increased osmolytes' consumption restores cell hydration, thus normalizing dry skin to normal well-moisturized skin condition. The main osmolytes, functioning in skin, are betaine, inositol and taurine, and their transmitters are betaine/y-aminobutyric acid (GABA) transmitter (BGT1), sodium-dependent myoinositol transmitter (SMIT) and taurine transmitters (TAUT), correspondingly. In response to hypertension, transportation of betaine increases, which is accumulated inside keratinocytes with further osmotic water flow, which restores cell volume. This is an important regulating mechanism of skin, so, to maintain water balance and homeostasis of physiological processes, cells are able to accumulate and release osmolytes in response to various external impacts. Consequently, betaine is an important epidermal osmolyte, required to maintain hydration of keratinocytes in dry environment or in exposure to UV-radiation.

It was demonstrated that protein composition is effectively osmophobic, and osmolytes can provide significant protein stability indirectly [https://www.ulprospector.com/en/eu/PersonalCare/Detail/6817/37783 8/GENENCARE-OSMS-BA-natural-betaine]. Osmolytes remove water from the first solvation shell of protein, allowing protein to fold and hide its basis. The ability of osmolytes, such as betaine, to increase protein folding motivity is caused by solvophobic effect on peptide backbone, exposed in deployed state. Another feature of these molecules is that they make the deployed state of macromolecules, such as proteins, in osmolytes' solution very unfavorable in comparison with folded state, not changing protein fold rules, occurring in dilute solutions. Osmolytes operate with increasing Gibbs free energy (ΔG) between naturally folded and deployed structures, thus shifting balance of folding to the side of naturally folded conformations. Thus, osmolytes, attracting water, increase thermodynamic stability of native folded state of proteins, but without direct interaction with protein, without interrupting cellular processes and without damaging cellular structures.

Additionally, trimethylglycine protects protein molecules and DNA from negative factors and allowing them functioning longer in skin. Adding 10 mM trimethylglycine stimulates cell differentiation and proliferation, protects DNA molecules from UV-damage [Rauhala L., Hämäläinen L., Dunlop T.W. et al. The organic osmolyte betaine induces keratin 2 expression in rat epidermal keratinocytes - A genome-wide study in UVB irradiated organotypic 3D cultures // Toxicology in Vitro, 2015, 30(1), 462-475].

However, nothing is known about long-term effect of betaine on pH-sensitive regulation of the number of Type 3 aquaporins in epidermal cells and on increase of transepidermal water flow in upper skin layers, which emphasizes the necessity of development of innovative complex for regulating long-term moisturizing mechanism of cell renew, supporting skin barrier function.

Patent RU 2456977C1 [V.I. Demenko, V.P. Scherban], published on July 27, 2012, describes cosmetic product for dry and very dry skin based on ceramide complex and various cosmetic additives. The invention relates to aesthetic medicine, or rather to cosmetology, and can be used as a product for aging skin care. The invention contains ceramide complex, anti-irritant complex, Langerhans cells activator, hyaluronic acid synthesis activator, natural moisturizing factor, as well as other cosmetically acceptable ingredients. For anti-irritant complex, Mediterranean aloe gel can be used in combination with other plant extracts. In composition of the natural moisturizing factor, a mixture of betaine with other acids can be used. The cosmetic product has high biological activity, stabilizes skin moisture content, has high lifting and rejuvenating effect.

The above-described composition from Patent RU 2456977C1, under the Authors' application, increases and stabilizes skin moisture content; however, it has complex multi-agent composition. The composition includes moisturizing agents, glycerin and urea, occlusal agents from silicone group, hyaluronic acid activator, amino acid derivatives; however, there are no examples with the results of the composition's impact on increasing the amount of Type 3 aquaporins in epidermal cells. The composition may have any other mechanism of action thanks to a large number of cosmetically acceptable substances with known effects on skin cells. The main action mode is creation on the surface of keratinous scales (reborn keratinocytes) a similarity of polylamellar layer with the composition of ceramides, which practically correspond to the same layer in skin; i.e., the composition is designed to eliminate the consequences of decreased moisture content, but non to eliminate the reason and the impact on natural processes in epidermal cells. The impact on this action mode provides strengthening the epidermal corneal layer, but does not significantly increase the moisture content, does not stimulate transepidermal water flow in cells and does not stimulate AQP3 generation to stimulate original cell update of keratinocytes, wound healing, moisture transportation in all epidermal layers. The examples do not specify which method was used to measure skin moisture content within clinical studies, which limits data interpretation.

Patent RU 2643928C2 [Pharmaceutical Reagents Institute Repharm Ltd.] published on February 06, 2016, describes health and beauty product for external use, related to pharmaceutical industry. The product includes a composition of disodium pyrophosphate and tetrapotassium pyrophosphate mixture in 1: 1 weight ratio and bisphosphonate; lipids, emulsifiers, structure-forming agents, biologically active and health supplements, flavorants, coloring agents, preservatives and water in a specific weight ratio of the mixture components (weight %), described in the patent. For biologically active and health supplements, various components can be used, including natural betaine, aloe vera gel. The invention provides decrease of crystallization rate of calcium slightly soluble salts in skin cells, thus eliminating calcination in the epidermal basal layer.

The above-described composition from Patent RU 2643928C2, under the Authors' application, decreases crystallization of calcium slightly soluble salts; however, it has complex multi-agent composition. The main active substances are potassium pyrophosphates and sodium pyrophosphates, as well as bisphosphonate, having high affinity to calcium ions. The use of the composition allows regulating mineral cell metabolism and decelerate skin cell aging process; however, the patent does not describe the use of the composition for skin moisturizing, pH-dependent stimulation of the amount of Type 3 aquaporins in skin and regulation of transepidermal water flow in skin cells. The impact on this action mode allows normalizing calcium metabolism, as well as eliminating calcificates in skin when aging, but does not significantly increase moisture content, does not stimulate transepidermal water flow in cells and does not stimulate AQP3 generation to stimulate original cell update of keratinocytes, wound healing, moisture transportation in all epidermal layers. The examples do not specify changes of moisture content in skin, Type 3 aquaporins stimulation within clinical studies, which limits data interpretation

Patent EA02619B1 [Zambon] published on March 31, 2017, describes local vaginal composition in the form of a gel, containing cows' colostral milk fraction, presented in free form and in the form of incapsulated mucoadhesive microspheres. The invention relates to local vaginal compositions in the form of gel, containing immune response mediators, growth factors, chemotactic factors and antibacterial, antiviral factors, extracted from cows' colostral milk and, not necessary, other ingredients with additional activity. The composition may additionally contain various ingredients, including betaine (1-10 weight %) and Aloe vera extract (1-5 weight %). The composition allows restoring and maintaining protective features of vagina, restore mucous layer on the surface, eliminate dry skin, caused by various factors. The composition quickly releases a part of active ingredients and a party of ingredients based on slow-release system to create long-term therapeutic effect, covering the period between further administrations.

The above-described composition from Patent EA02619B1 contains various groups of biologically active substances, including pharmaceuticals substances. The main application method is elimination of dry vagina, which is different from other human skin areas in its physiological and biochemical properties. The administration method determines the composition and the selection of biologically active substances. The use of the composition allows restoring vaginal mucus membrane and natural protective factors; however, the patent does not describe the use of the composition for skin moisturizing, pH-dependent stimulation of the amount of Type 3 aquaporins in skin and regulation of transepidermal water flow in skin cells. It is known that the vaginal mucus membrane does not contain Type 3 aquaporins in the layers, and moisturizing is achieved by increased mucus secretion and its adhesion on mucus membranes. The examples do not specify measuring moisture content in skin, stimulating Type 3 aquaporins within clinical studies; they specify only estimation of dry vagina after long-term used of the gel by fertile women in the period of perimenopause and menopause. It is known that later, dry vagina, caused by hormonal or mechanical reasons, stress, can be eliminated by endocrine profile normalization; this is why, the composition has preventive action and decreases the severity of dry vagina.

Each of the components of the invention individually have been used in the production of cosmetic products.

Shower gel "Estel Haute Couture Hydrobalance" (<URL: https://flowwow.com/cosmetics-perfume/gel-dlya-dusha-luxury-hydrobal/>) is known, containing the following components (INCI names are specified): Sodium Chloride, Parfum, Limonene, Citral, Linalool, Betaine, Aloe Barbadensis Leaf Juice, Sodium Benzoate, Polyquaternium-7, Polyquaternium-10, Glycerin, Propylene Glycol, Sorbitol, Panthenol, Carboxymethyl Chitin, Sodium Hyaluronate, Citric Acid, Tetrasodium EDTA, Methylchloroisothiazolinone, Methylisothiazolinone. The known shower gel contains Betaine, Aloe Barbadensis Leaf Juice.

Bath and shower gel «Petal Fresh Aloe & Citrus» (<URL: https://www.biggreensmile.com/products/petal-fresh-refreshing-bath-shower-gel-aloe-citrus/ptshowaloec.aspx?productid=ptshowaloec>) is known, containing the following components (INCI names are specified): Aqua (water), betaine (derived from sugar beets), sodium lauroyl methyl isethionate (derived from coconut), sodium cocoyl isethionate (derived from coconut oil), ammonium cocoyl isethionate (derived from coconut oil), hydroxypropyl methylcellulose (derived from cotton fiber), ^{∗}aloe barbadensis (aloe vera) leaf juice, ^{∗}citrus aurantium dulcis (orange) fruit extract, ^{∗}citrus limon (lemon) fruit extract, ^{∗}citrus grandis (grapefruit) leaf extract, ^{∗}citrus nobilis (mandarin orange) fruit extract, orbignya oleifera (babassu) seed oil, tocopheryl acetate (vitamin E), caprylic/capric triglyceride (derived from coconut oil), panthenol (vitamin B5), glycerin (derived from vegetable oil), ^{∗}tussilago farfara (coltsfoot) flower extract, ^{∗}achillea millefolium (yarrow) extract, ^{∗}equisetum arvense (horsetail) extract, ^{∗}rosmarinus officinalis (rosemary) leaf extract, ^{∗}althaea officinalis (marshmallow) root extract, ^{∗}chamomilla recutita (chamomile) flower extract, ^{∗}melissa officinalis (lemon balm) leaf extract, ^{∗}thymus vulgaris (thyme) extract, polysorbate-20 (derived from coconut oil), citric acid (derived from citrus fruit), dehydroacetic acid (derived from cane sugar), ethylhexylglycerin (derived from vegetable oil), sodium chloride (derived from salt), ^{∗∗}fragrance, where *Certified organic ingredients, ^{∗∗}Natural fragrance with essential oils. The known bath and shower gel contains Betaine, Aloe Barbadensis Leaf Juice.

Shower gel «Fa» «Aloe Vera Yogurt» (<URL: https://www.amazon.co.uk/Fa-Shower-Yogurt-Aloe-Vera/dp/B01DUF0Y4S>) is known, containing the following components (INCI names are specified): Aqua - Sodium Laureth Sulfate - Cocamidopropyl Betaine - Sodium Chloride - Yogurt - Aloe Barbadensis Leaf Juice - Niacinamide - Parfum - Polyquaternium-7 - Propylene Glycol - PEG-7 Glyceryl Cocoate - PEG-55 Propylene Glycol Oleate - Laureth-2 - Styrene/Acrylates Copolymer - Tetrasodium EDTA - BHT - Citric Acid - Geraniol - Benzyl Alcohol - Hexyl Cinnamal - Butylphenyl Methylpropional - Citronellol - Sodium Benzoate - Sodium Salicylate. The known shower gel contains Aloe Barbadensis Leaf Juice.

Shower gel Topicream DA (<URL: https://www.amazon.com/Topicrem-Atopic-Ultra-Rich-Cleansing-500ml/dp/B00PTZXTQ8 >) is known, containing the following components (INCI names are specified): Aqua (water), Glycerin, Sodium Laureth Sulfate, Betaine, Coco-Glucoside, Glyceryl Oleate, PEG-200 Hydrogenated Glyceryl Palmate, Sodium Cocoamphoacetate, 1,2-Hexanediol, PEG-7 Glyceryl Cocoate, Linum Usitatissimum (Linseed) Seed Oil, Citric Acid, Allantoin, Sodium Chloride, Ascorbyl Palmitate, Hydrogenated Palm Glycerides Citrate, Sodium Sulfate Lecithin, Tocopherol. The known shower gel contains *Betaine.*

As opposed to Patents RU2456977C1, RU2643928C2, EA02619B1, the present inventors studied compositions of shower gels, soap, shampoos and determined the efficiency of compound composition of N-trialkylated glycine derivative, in particular, trimethylglycine, and specifically *Aloe Barbadensis* leaf extract and their synergistically positive effect on skin condition, in particular, pH-dependent increase of the amount of Type 3 aquaporins and regulation of transepidermal water flow in epidermal cells of body skin and scalp. *Aloe Barbadensis Leaf Juice* assumes a different composition of biologically active substances, different from *Aloe Barbadensis* leaf extract, according to data from scientific literature.

The technical result of innovative complex is stimulation of the amount of Type 3 aquaporins to maintain natural skin moisture level and simultaneous redistribution of transepidermal water flow in all epidermal layers to decrease TEWL, which is manifested in improvement of sensitive and atopic skin condition. The complex is active in the range of pH 4.5-7.5, which corresponds to most body areas and allows performing pH-dependent regulation of AQP3.

It is found out that combination *of Aloe Barbadensis* leaf extract and N-trialkylated glycine derivative, in particular, trimethylglycine of natural origin, allows increasing the amount of AQP3 proteins in all epidermal layers and regulating transepidermal water flow between cells of the whole epidermis to decrease TEWL in dehydrated skin. The use of the complex helps reducing transepidermal water loss, increasing the amount of Type 3 aquaporins in all epidermal layers for long-term prevention of dry skin, caused by specific impact of various environmental factors and the use of unsuitable cosmetic ingredients in the product composition. pH-dependent regulation of Type 3 aquaporins allows maintaining pH-gradient of Marcionini's mantle and buffer capacity of the epidermal corneal layer and maintain skin barrier layer, as well as stimulating deep water flow in all epidermal layer cells. According to the data from scientific literature, long-term increase of the number of AQP3 allows preventing dermatological skin disorders, such as atopic dermatitis, eczema, psoriasis and vitiligo.

Joining the components allows reaching synergetic effect referring to aquaporin number of AQP3 and preserving the efficiency in various percent of administration of individual components. The synergetic effect is expressed in stimulation of AQP3 aquaporin number in all epidermal layers and upper dermal layer, long-term regulation of pH-gradient of acid mantle on skin surface, decrease of TEWL and increase of deep moisture content. *Aloe Barbadensis* leaf extract stimulates synthesis and integration of AQP3 to epidermal cell membrane in deep layers, protects them from negative environmental factors and trimethylglycine provides water transportation properties of cells of skin itself and of upper epidermal layer for deep penetration, stabilizes AQP3 native structure in cell membranes and changes viscoelastic-plastic properties of the epidermal corneal layer. Thanks to the use of the complex, cell hydration is increased sue to natural osmolytes in the complex, osmotic gradient in epidermal cells and stabilizes AQP3 folding to naturally folded aquaporin conformations for specific transportation of water flow. The complex does not interfere with cell processes and does not damage cell structures during the action; it impacts gene deep moisturizing processes in skin itself, does not make occlusion film on cell surface and stimulates water flow in all skin layers. Epidermal cells preserve their ability of cellular renewal, maintenance of barrier function and transepidermal water flow in deep layers.

An additional feature is decrease of transepidermal water, hypoallergenicity and safe use of cosmetic products, containing the biocomplex, in patients with various allergic disorders, decrease of complex allergenicity index in patients with various dermatological skin disorders (recurrent urticaria, atopic dermatitis, chronic contact dermatitis, eczema, itching and rhinorrhea). Increase of AQP3 number and regulation of transepidermal water flow favors the restoration of skin barrier layer, maintenance of pH-gradient at the physiological level, as well as improvement of hair condition and moisturizing of scalp. Additionally, the biocomplex based on these ingredients is 100% natural, it is biodegradable and complies with the requirements of voluntary ecological certification: Vegan, Biorius Natural, Cosmos Ecocert.

### SUMMARY OF THE INVENTION

In the first aspect of invention, the invention relates to the biocomplex for body skin and scalp care, in particular, for pH-dependent regulation of Type 3 aquaporins and distribution of transepidermal water flow in body skin and scalp cells. The invention relates to the biocomplex and comprises of A and B components, where
(A) Plant extraction from Aloe Barbadensis, in particular, Aloe Barbadensis leaf extract;
(B) N-trialkylated glycine derivative, in particular, trimethylglycine or betaine,
   the raw material ratio equivalent to active ingredients of the components (A) and (B) as per wt.% is (0.0001-1.00):(0.1-10).
   and wherein the (A):(B) raw material ratio is
      - higher 1:5, preferably higher 1:6, further preferably higher 1:7; further preferably higher 1:8, further preferably higher 1:9, further preferably higher 1:10; or
      - lower 1:5, preferably lower 1:6, further preferably lower 1:7; further preferably lower 1:8, further preferably lower 1:9, further preferably lower 1:10.

The raw materials are resources of mineral, animal or plant origin that are used for cosmetics or other products development or used to get individual active substance. These raw materials can have comprehensive composition, additives, and special components. For example, Aloe Barbadensis leaves are used to get the different extract, individual and purified biological active substances. Another example is water-glycerol extract of Aloe barbadensis contains plant extract of Aloe Barbadensis leaves, water, glycerol, preservative and pH regulator where only Aloe barbadensis extract is active ingredient and defines biological effect.

The active ingredients are specific purified substances of mineral, animal or plant origin that are contained in raw materials and have clear physiological effect on skin, oral cavity, hair or other part of body, with established biological activity on any target (enzymes, receptors, cellular process etc). These active ingredients can include humectants, thickening agents, film formers, ultraviolet absorbents, antioxidants, sequestering agents, vitamins, pharmaceutical agents such as plant extracts, perfume or other components. For example, Aloe Barbadensis extract is active ingredient with clear biological effect on aquaporines AQP3 and therefore on skin moisturising effect as was established by Authors of invention. The composition is different in that plant extraction from *Aloe Barbadensis* is a substance or commercial plant *Aloe Barbadensis* leaf extract with Registration Number CAS 85507-69-3 or 94349-62-9. Plant extraction from *Aloe Barbadensis* leaves can be *Aloe Barbadensis* leaf extract, such as Botanical Aloe Gly (kbA) or Aloe Vera Gel 10x1 or Aloe extract (item 204-415) Groumant or other commercially available raw material items.

For the present invention, *Aloe Barbadensis* leaf extract can be dry extract, aqueous extract, alcoholic extract, hydroalcoholic extract, hydroglyceric extract, oily extract or CO₂-extract; preferably, hydroglyceric or dry extract.

The composition is different in that N-trialkylated glycine derivative is a substance or commercially available trimethylglycine or betaine with Registration Number CAS 107-43-7. N-trialkylated glycine derivative can be trimethylglycine or betaine, such as Trimethylglycine Genencare OSMS BA or other commercially available raw material items.

For the invention, the composition may include active ingredients, weight %:

| | |
|---|---|
| *Aloe Barbadensis* leaf extract | 0.0001-1.00%, |
| N-trialkylated glycine derivative, trimethylglycine | 0.10-10.00% |

In the second aspect, the invention relates to shower gel, containing 0.1001-11.00% composition for the invention.

In the third aspect, the invention relates to liquid hand wash, containing 0.1001-11.00% composition for the invention.

In the fourth aspect, the invention relates to hair and scalp shampoo, containing 0.1001-11.00% composition for the invention.

In the fifth aspect, the invention relates to anti-dandruff, seborrheic and xerosis shampoo, containing 0.1001-11.00% composition for the invention.

In the sixth aspect, the invention relates to hair balm or hair conditioner, containing 0.1001-11.00% composition for the invention.

In the seventh aspect, the invention relates to face wash products, such as cleansing gel, cleansing gel scrub, cleansing mousse foam, micellar water, containing 0.1001-11.00% composition for the invention.

In the eighth aspect, the invention relates to face, body, hand cream, containing 0.1001-11.00% composition for the invention.

In the ninth aspect, the invention relates to face, scalp serum, containing 0.1001-11.00% composition for the invention.

In the tenth aspect, the invention relates to solid products for face, hand and hair care, such as solid bar soap, cream soap, solid shampoo, solid balm, solid conditioner, containing 0.1001-11.00% composition for the invention.

In the eleventh aspect, the invention relates to the use of the composition for the invention for pH-dependent regulation of Type 3 aquaporins and distribution of transepidermal water flow in body and scalp skin cells.

### DETAILED SPECIFICATION

The claimed invention relates to the biocomplex for body skin and scalp care, in particular, for pH-dependent regulation of Type 3 aquaporins and distribution of transepidermal water flow in body skin and scalp cells, containing the combination of Aloe Barbadensis: trimethylglycine raw material ratio equivalent to active ingredients is (0.0001-1.00):(0.1-10.0) weight %.

*Aloe Barbadensis* leaf extract (INCI: Aloe Barbadensis Leaf Extract) is a commercially available ingredient of cosmetic products, well-known by persons skilled in the art.

N-trialkylated glycine derivative, in particular, trimethylglycine (INCI: Betaine) is a commercially available ingredient of cosmetic products, well-known by persons skilled in the art.

In the product for the invention, *Aloe Barbadensis* leaf extract can be dry extract, aqueous extract, alcoholic extract, hydroalcoholic extract, hydroglyceric extract, oily extract or CO2-extract; preferably, hydroglyceric or dry extract. The specified extracts are well-known by persons skilled in the art and are commercially available from various suppliers.

In the product for the invention, *Aloe Barbadensis* leaf extract can be aqueous *Aloe Barbadensis* leaf extract, with an addition of a preservative, sodium benzoate or potassium sorbate, with or without addition of glycerin and pH regulator, citric acid.

The composition for the invention may contain active ingredients, weight %:

| | |
|---|---|
| *Aloe Barbadensis* leaf extract | 0.0001-1.00%, |
| N-trialkylated glycine derivative, trimethylglycine | 0.10-10.00% |

In the product for the invention, acceptable excipients can be selected from the following categories of components.

Anionic surfactants:
1. Amide salt of higher fatty acid and metylglycin with the general formula R4-C(O)-N(-CH3)-CH2-CO2X4, where R4 is alkene and/or alkenyl group with hydrocarbon-based chain length from 5 to 21 carbon atoms, and X4 is a cation of alkali metal and/or alkali-earth metal, ammonium, alkylammonium, alkanolammonium, glucoammonium;
2. Alkyl polyethylene glycol carboxylate with the general formula: RS-O(-CH2-CH2-O-)n2CH2-CO2X5, where n2 can accept values from 1 to 15, and means the number of polyethyleneglycol groups, R5 - alkene and/or alkenyl group with hydrocarbon-based chain length from 6 to 22 carbon atoms, and X5 is a cation of alkali metal and/or alkali-earth metal, ammonium, alkylammonium, alkanolammonium, glucoammonium;
3. Twice-substituted salt of 2-sulphocarboxylic acid with the general formula: R6-CH(-SO3X6)-CO2X6, where R6 is alkene and/or alkenyl group with hydrocarbon-based chain length from 4 to 20 carbon atoms, and X6 is a cation of alkali metal and/or alkali-earth metal, ammonium, alkylammonium, alkanolammonium, glucoammonium;
4. Mono- or twice-substituted salt of higher carboxylic acid amide and glutamic acid amide with the general formula: R7-C(O)-NH-CH(-CH2-CH2-CO2X7)-CO2X7, where R7 is alkene and/or alkenyl group with hydrocarbon-based chain length from 5 to 21 carbon atoms, and X7 is a cation of alkali metal and/or alkali-earth metal, ammonium, alkylammonium, alkanolammonium, glucoammonium or hydrogen;
5. Amide salt of higher fatty acid and glycine with the general formula: R8-C(O)-NH-CH2-CO2X8, where R8 is alkene and/or alkenyl group with hydrocarbon-based chain length from 5 to 21 carbon atoms, and X8 is a cation of alkali metal and/or alkali-earth metal, ammonium, alkylammonium, alkanolammonium, glucoammonium;
6. Amide salt of higher fatty acid and alanine with the general formula: R9-C(O)-NH-CH(-CH3)-CO2X9, where R9 is alkene and/or alkenyl group with hydrocarbon-based chain length from 5 to 21 carbon atoms, and X9 is a cation of alkali metal and/or alkali-earth metal, ammonium, alkylammonium, alkanolammonium, glucoammonium;
7. Amide salt of higher fatty acid and 2-aminomethylethanesulfonic acid with the general formula: R10-C(O)-N(-CH3)-CH2-CH2-SO3X10, where R10 is alkene and/or alkenyl group with hydrocarbon-based chain length from 5 to 21 carbon atoms, and X10 is a cation of alkali metal and/or alkali-earth metal, ammonium, alkylammonium, alkanolammonium, glucoammonium;
8. Alkylpolyglucoside hydroxypropylsulphonate with the general formula: R11-O-[G]p1-O-CH2-CH(-OH)-CH2-SO3X11, where R11 is alkene and/or alkenyl group with hydrocarbon-based chain length from 6 to 22 carbon atoms, G is a saccharide fragment, containing 5 or 6 carbon atoms, p1 can accept values from 1 to 4, and X11 is a cation of alkali metal and/or alkali-earth metal, ammonium, alkylammonium, alkanolammonium, glucoammonium;
9. Alkylpolyglucoside carboxylate with the general formula: R12-O-[G]p2-O-CH2-CO2X12, where R12 is alkene and/or alkenyl group with hydrocarbon-based chain length from 6 to 22 carbon atoms, G is a saccharide fragment, containing 5 or 6 carbon atoms, p2 can accept values from 1 to 4, and X12 is a cation of alkali metal and/or alkali-earth metal, ammonium, alkylammonium, alkanolammonium, glucoammonium;
10. Amide salt of higher fatty acid and threonine with the general formula: R13-C(O)-NH-CH(-CH(-OH)-CH3)-CO2X13, where R13 is alkene and/or alkenyl group with hydrocarbon-based chain length from 5 to 21 carbon atoms, and X13 is a cation of alkali metal and/or alkali-earth metal, ammonium, alkylammonium, alkanolammonium, glucoammonium;
11. Amide salt of higher fatty acid and amino acid, received by plant-based protein hydrolysis, with the general formula: R14-C(O)-AAX14, where R14 is alkene and/or alkenyl group with hydrocarbon-based chain length from 5 to 21 carbon atom, AA - amino acid or peptide, received by plant-based protein hydrolysis (possible protein sources are apples, soya, wheat, cotton etc.), and X14 is a cation of alkali metal and/or alkali-earth metal, ammonium, alkylammonium, alkanolammonium, glucoammonium.

Amphoterous surphactants:
1. Twice-substituted acylamphodiacetate salt with the general formula: R15-C(O)-NH-CH2-CH2-N(-CH2-CO2X15)-CH2-CH2-O-CH2-CO2X15, where R15 is alkene and/or alkenyl group with hydrocarbon-based chain length from 5 to 21 carbon atoms, and X15 is a cation of alkali metal and/or alkali-earth metal, ammonium, alkylammonium, alkanolammonium, glucoammonium;
2. Acylamphoacetate salt with the general formula: R16-C(O)-NH-CH2-CH2-N(-CH2-CO2X16)-CH2-CH2-OH, where R16 is alkene and/or alkenyl group with hydrocarbon-based chain length from 5 to 21 carbon atoms, and X16 is a cation of alkali metal and/or alkali-earth metal, ammonium, alkylammonium, alkanolammonium, glucoammonium;
3. Alkylamphoacetate salt with the general formula: R17-C(=N-CH2-CH2-N((-CH2-CH2-OH)-CH2-CO2X17)-), where R17 is alkene and/or alkenyl group with hydrocarbon-based chain length from 5 to 21 carbon atoms, and X17 is a cation of alkali metal and/or alkali-earth metal, ammonium, alkylammonium, alkanolammonium, glucoammonium;
4. Acylamidoalkylbetaine with the general formula: R18-C(O)-NH-R19-N(-CH3)2)-CH2-CO2 , where R18 is alkene and/or alkenyl group with hydrocarbon-based chain length from 5 to 21 carbon atoms, R19 is alkene group with hydrocarbon-based chain length from 1 to 4 carbon atoms;
5. Acylamidoalkylhydroxysultaine with the general formula: R20-C(O)-NH-R21-N(-CH3)2-CH2-CH(-OH)-CH2-SO3, where R20 is alkene and/or alkenyl group with hydrocarbon-based chain length from 5 to 21 carbon atoms, R21 is alkene group with hydrocarbon-based chain length from 1 to 4 carbon atoms;
6. Acydamidoalkylamine oxide with the general formula: R22-C(O)-NH-R23-N(-CH3)2-O, where R22 is alkene and/or alkenyl group with hydrocarbon-based chain length from 5 to 21 carbon atoms, R23 is alkene group with hydrocarbon-based chain length from 1 to 4 carbon atoms;
7. Alkylbetaine with the general formula: R24-N(-CH3)2)-CH2-CO2, where R24 is alkene and/or alkenyl group with hydrocarbon-based chain length from 5 to 21 carbon atoms;
8. Alkylhydroxysultaine with the general formula: R25-N(-CH3)2-CH2-CH(-OH)-CH2-SO3, where R25 is alkene and/or alkenyl group with hydrocarbon-based chain length from 6 to 22 carbon atoms;
9. Alkylsultaine with the general formula: R26-N(-CH3)2-CH2-CH2-CH2-SO3, where R26 is alkene and/or alkenyl group with hydrocarbon-based chain length from 6 to 22 carbon atoms;
10. Alkylamine oxide with the general formula: R27-N(-CH3)2-O, where R26 is alkene and/or alkenyl group with hydrocarbon-based chain length from 6 to 22 carbon atoms.

Non-ionogenic surfactants:
1. Alkylpolyethyleneglycol with the general formula: R29-O(-CH2-CH2-O-)n3H, where n3 can accept values from 2 to 20, and means the number of polyethyleneglycol groups, R29 - alkene and/or alkenyl group with hydrocarbon-based chain length from 6 to 22 carbon atoms;
2. Alkylpolyethylene/propyleneglycol with the general formula: R30-O(-CH2-CH2-O-)n4(-CH(-CH3)-CH2-O-)n5H, where n4 can accept values from 2 to 20, and means the number of polyethyleneglycol groups, n5 can accept values from 2 to 20, and means the number of propyleneglycol groups, R30 - alkene and/or alkenyl group with hydrocarbon-based chain length from 6 to 22 carbon atoms;
3. Dialkylpolyethyleneglycol with the general formula: R31-O(-CH2-CH2-O-)n6R32, where n6 can accept values from 2 to 20, and means the number of polyethyleneglycol groups, R31 is alkene and/or alkenyl group with hydrocarbon-based chain length from 6 to 22 carbon atoms, R32 is alkene and/or alkenyl group with hydrocarbon-based chain length from 1 to 12 carbon atoms;
4. Dialkylpolyethylene/propyleneglycol with the general formula: R33-O(-CH2-CH2-O-)n7(-CH(-CH3)-CH2-O-)n8-R34, where n7 can accept values from 2 to 20, and means the number of polyethyleneglycol groups, n8 can accept values from 2 to 20, and means the number of propyleneglycol groups, R33 is alkene and/or alkenyl group with hydrocarbon-based chain length from 6 to 22 carbon atoms, R34 is alkene and/or alkenyl group with hydrocarbon-based chain length from 1 to 12 carbon atoms.

Dispersion medium for polysaccharide / solvent:
1. Organic alcohol with the general formula: R35(-OH)s1, where R35 is alkene group with hydrocarbon-based chain length from 3 to 12 carbon atoms, S1 can accept values from 1 to 12, and means the number of hydroxyl groups, located in the hydrocarbyl randomly to each other;
2. Alkylpolypropyleneglycol with the general formula: H(-CH(-CH3)-CH2-O-)n9R36, where n9 can accept values from 2 to 10, and means the number of propyleneglycol groups, R36 is alkene group with hydrocarbon-based chain length from 1 to 10 carbon atoms.

pH regulators:
1. Organic acids with the general formula: R37(-OH)s2(-COOH)m1, where R37 is alkene group with hydrocarbon-based chain length from 1 to 12 carbon atoms, S2 can accept values from 1 to 12, and means the number of hydroxyl groups, located in the hydrocarbyl, randomly to each other, M1 can accept values from 1 to 4, and means the number of carboxyl groups, located in the hydrocarbyl, randomly to each other;
2. Solutions of alkali or alkali-earth metal hydroxides, ammonia, primary and tertiary alkylamines, primary and tertiary alkanolamines, primary and tertiary glucamines, basic amino acids, citric acid disodium salt, citric acid trisodium salt.

Chelating agent:
1. Metylglycindiacetic acid trisodium salt, glutaminediacetic acid tetrasodium salt, ethylendiamine-(N,N)-disuccinate trisodium salt.;
2. Organic acids and alkali metal salts, ammonium, alkylammonium, alkanolammonium, glucoammonium, corresponding to the following acids: citric acid, apple acid, tartaric acid, glutaric acid, adipic acid, glucuronic acid, galacturonic acid, galactaric acid, gluconic acid, phytic acid, polytaconic acid, polyacrylic acid, polymethacrylic acid, copolymer of acrylic and maleic acids, as well as organic acids with the general formula R38(-OH)s3(-COOH)m2, where R38 is alkene group with hydrocarbon-based chain length from 1 to 12 carbon atoms, S3 can accept values from 1 to 12, and means the number of hydroxyl groups, located in the hydrocarbyl, randomly to each other, M2 can accept values from 1 to 4, and means the number of carboxylic group, located in the hydrocarbyl, randomly to each other.

Preservatives:
1. Organic acids and alkali and alkali-earth metal salts, ammonium, alkylammonium, alkanolammonium, glucoammonium, corresponding to the following acids: benzoic acid, sorbic acid, 4-methoxybenzoic acid, salicylic acid, undecylenic acid;
2. Organic alcohol and phenols: phenoxyethanol, benzyl alcohol, caprylyl glycol, ethylhexylglycerin, phenethyl alcohol, 3-methyl-4-isopropylphenol, 2,4-dichlorobenzyl alcohol;
3. Biocides of broad spectrum: benzisothiazolinone, methylisothiazolinone, dodecyldipropylene triamine;
4. Fungicides: sodium pyrithione, climbazole.

Fragrances with or without essential oils of any olfactory profile, designed to confer improved consumer properties.

Essential oils in pure form or in the form of mixtures in different ratios: orange, bergamot, lemon, lime, tangerine, grapefruit, neroli, rosewood, yuzu, lemongrass, lavender, sage, thyme, lemon balm, mint of various botanical species, tea tree, eucalyptus, cedar, sandalwood, black pepper, pink pepper, cinnamon , cardamom, coriander, jasmine, rose, peony, blue chamomile, basil, geranium, as well as any other commercially available essential oils, known by persons skilled in the art.

### EXPERIMENTAL DATA

Examples, introduced into the present description, are not limiting for the claimed invention and are given in order to demonstrate and confirm the achievement of the expected technical results. These examples are ones out of many experimental data, received by the present inventors, which confirm the effectiveness of products within the scope of the invention.

### Example 1.

Components to be introduced to the composition for the invention have been studied in the composition of various cosmetic products

Components to be introduced to the composition for the invention have been studied in the composition of various cosmetic products. A liquid product for body wash was prepared, in particular, shower gel, under the scope of the present invention (Table No. 1).

**Table No. 1. Formulation of the shower gel with the claimed composition**

| **Item No.** | **Component** | **Content, weight %** |
|---|---|---|
| 1 | Purified water | Max. 100 |
| 2 | Sodium coco-sulfate | 3.5-10.0 |
| 3 | Decyl glucoside | 3.0-10.0 |
| 4 | Cocamidopropylbetaine 40-45% | 5.0-10.0 |
| 5 | Glutamate diacetate tetrasodium salt | 0.05-0.4 |
| 6 | Sodium chloride | 0.1-0.4 |
| 7 | Monohydrate citric acid | 0.1-0.5 |
| 8 | Potassium sorbate, sodium benzoate | 0.2-1.0 |
| 9 | Trimethylglycine | 0.1-10.0 |
| 10 | *Aloe Barbadensis* leaf extract | 0.0001-1.0 |
| 11 | Fragrance with components of natural origin | 0.0-0.8 |
| 12 | Essential oil, or a combination of essential oils | 0.0-0.8 |
| 13 | Aqueous solution of citric acid and silver citrate | 0.0-0.2 |

The prepared shower gel provides high cleansing capacity, foam formation and foam elasticity, has a hypoallergic effect as well as regulates pH of the body skin, increases the amount of Type 3 aquaporins, assists in distribution of transepidermal water flow in body skin cells and moisturizes the body skin.

### Example 2.

Components to be introduced to the composition for the invention have been studied in the composition of various cosmetic products. A liquid body and hair wash was prepared, in particular, hair and body wash, under the scope of the present invention (Table No. 2).

**Table No. 2. Formulation of the hair and body wash with the claimed composition**

| **Item No.** | **Component** | **Content, weight %** |
|---|---|---|
| 1 | Purified water | Max. 100 |
| 2 | Sodium coco-sulfate | 3.5-10.0 |
| 3 | Decyl glucoside | 3.0-10.0 |
| 4 | Cocamidopropylbetaine 40-45% | 5.0-10.0 |
| 5 | Glutamate diacetate tetrasodium salt | 0.05-0.4 |
| 6 | Sodium chloride | 0.1-0.4 |
| 7 | Glycerin | 0.1-3.0 |
| 8 | Conditioning agent | 0.1-0.5 |
| 9 | Monohydrate citric acid | 0.1-0.5 |
| 10 | Potassium sorbate, sodium benzoate | 0.2-1.0 |
| 11 | Trimethylglycine | 0.1-10.0 |
| 12 | *Aloe Barbadensis* leaf extract | 0.0001-1.0 |
| 13 | Fragrance with components of natural origin | 0.0-0.8 |
| 14 | Essential oil, or a combination of essential oils | 0.0-0.8 |
| 15 | Aqueous solution of citric acid and silver citrate | 0.0-0.2 |
| 16 | Coloring agent | 0.00001-0.5 |

The prepared hair and body wash provides high cleansing capacity, foam formation and foam elasticity, has a hypoallergic effect and a sebo-regulating effect, as well as regulates pH of the body skin and scalp, increases the amount of Type 3 aquaporins, assists in distribution of transepidermal water flow in body skin and scalp cells and moisturizes the body skin and the scalp.

### Example 3.

Components to be introduced to the composition for the invention have been studied in the composition of various cosmetic products. A liquid product for scalp cleansing and hair care was prepared, in particular, a hair shampoo, under the scope of the present invention (Table No. 3).

**Table No. 3. Formulation of the hair shampoo with the claimed composition**

| **Item No.** | **Component** | **Content, weight %** |
|---|---|---|
| 1 | Purified water | Max. 100 |
| 2 | Lauroyl sodium methylisothionate | 5.0-10.0 |
| 3 | Lauryl glucoside | 2.0-8.0 |
| 4 | Cocamidopropylbetaine 40-45% | 3.0-6.0 |
| 5 | Sodium cocoyl isethionate | 1.0-4.0 |
| 6 | Glutamate diacetate tetrasodium salt 47% solution | 0.1-0.8 |
| 7 | Glycerin | 0.1-3.0 |
| 8 | Guar hydroxypropyl trimonium chloride | 0.1-0.5 |
| 9 | Monohydrate citric acid | 0.1-0.5 |
| 10 | Preservative | 0.2-1.0 |
| 11 | Trimethylglycine | 0.1-10.0 |
| 12 | *Aloe Barbadensis* leaf extract | 0.0001-1.0 |
| 13 | Fragrance | 0.05-0.5 |
| 14 | Essential oil, or a combination of essential oils | 0.05-0.6 |
| 15 | Additional active substances designed for hair care | 0.1-5.0 |

The prepared hair shampoo provides high cleansing capacity, foam formation and foam elasticity, has a hypoallergic effect and a sebo-regulating effect, impacts thickening of hair fibers, hair strength, normalization of hair porosity, hair moisturizing, protection from thermal damage, as well as regulates pH of the scalp, increases the amount of Type 3 aquaporins, assists in distribution of transepidermal water flow in body skin and scalp cells and moisturizes the body skin and the scalp.

### Example 4.

Components to be introduced to the composition for the invention have been studied in the composition of various cosmetic products. A liquid hair care product was prepared, in particular, a hair balm, under the scope of the present invention (Table No. 4).

**Table No. 4. Formulation of the hair balm with the claimed composition**

| **Item No.** | **Component** | **Content, weight %** |
|---|---|---|
| 1 | Purified water | Max. 100 |
| 2 | Fatty alcohols C16-C18 | 3.5-7.0 |
| 3 | Cation surfactant based on quaternary ammonium compounds | 0.5-10.0 |
| 4 | Conditioning additive | 0.5-3.0 |
| 5 | Refined oils | 0.2-1.0 |
| 6 | Hydroxyethyl cellulose | 0.3-1.0 |
| 7 | Glycerin | 1.0-5.0 |
| 8 | Octyldodecanol | 0.2-2.0 |
| 9 | Trimethylglycine | 0.1-10.0 |
| 10 | *Aloe Barbadensis* leaf extract | 0.0001-1.0 |
| 11 | Amino acid complex | 0.1-0.5 |
| 12 | Glutamate diacetate tetrasodium salt 47% solution | 0.05-0.5 |
| 13 | Organic acid for pH regulation | 0.03-0.4 |
| 14 | Fragrance | 0.1-0.8 |
| 15 | Essential oil, or a combination of essential oils | 0.05-0.6 |
| 16 | Additional active substances designed for hair care | 0.2-4.0 |
| 17 | Coloring agent of natural origin | 0.01-0.1 |

The prepared hair balm has a hypoallergic effect, impacts thickening of hair fibers, hair strength, normalization of hair porosity, hair moisturizing, protection from thermal damage, as well as regulates pH of the scalp, increases the amount of Type 3 aquaporins, assists in distribution of transepidermal water flow in scalp cells, moisturizes and nourishes the scalp if used together with the hair and scalp shampoo.

### Example 5.

Components to be introduced to the composition for the invention have been studied in the composition of various cosmetic products. A liquid product for hand care was prepared, in particular, a liquid hand wash, under the scope of the present invention (Table No. 5).

**Table No. 5. Formulation of the liquid hand wash with the claimed composition**

| **Item No.** | **Component** | **Content, weight %** |
|---|---|---|
| 1 | Purified water | Max. 100 |
| 2 | Glycerin | 0.1-3.0 |
| 3 | Sodium coco-sulfate | 3.5-10.0 |
| 4 | Coco glucoside | 3.0-10.0 |
| 5 | Sunflower oil methylglucamide | 0.1-5.0 |
| 6 | Cocamidopropylbetaine 40-45% | 2.0-7.0 |
| 7 | Glutamate diacetate tetrasodium salt 47% solution | 0.05-0.4 |
| 8 | Lactic acid | 0.2-0.9 |
| 9 | Preservative | 0.2-1.0 |
| 10 | Hydro-glycerin cotton extract | 0.01-0.5 |
| 11 | *Aloe Barbadensis* leaf extract | 0.0001-1.0 |
| 12 | Trimethylglycine | 0.1-10.0 |
| 13 | Fragrance | 0.0-0.8 |
| 14 | Aqueous solution of citric acid and silver citrate | 0.0-0.2 |
| 15 | Other additional ingredients | 0.005-1 |

The prepared liquid hand wash provides high cleansing capacity, foam formation and foam elasticity, has a hypoallergic effect, as well as regulates pH of the hand skin, increases the amount of Type 3 aquaporins, assists in distribution of transepidermal water flow in hand skin cells, moisturizes the hand skin.

Pre-clinical and clinical studies were performed to estimate the efficiency and stability of the composition for the invention in formulation of various cosmetic products for the invention.

### Example 6.

An in vitro study was performed to determine the amount of Type 3 aquaporins in skin keratinocytes after using the components in the composition for the invention.

Aquaporins are proteins, forming pores in cells to regulate transepidermal water flow and low molecular substances, e.g., glycerin. Type 3 aquaporins (AQP3) are basically found in skin cells, as well as respiratory system and kidney cells to regulate water flow. The amount of AQP3 in the basal and superbasal layers directly determine the degree of water penetration to various skin layers to epidermis, normal differentiation and peeling of keratinocytes, as well as maintenance of skin barrier layer.

The study method is based on the definition of the amount of Type 3 aquaporins in epidermal keratinocytes after adding the components of the composition into HaCaT cell culture medium. At the first stage, cytotoxicity of the components is estimated depending on the concentration of components in the composition, be determining the number of survived cells according to MTT concentration with a color dilution method. Cells of HaCaT line were used in the amount of 10 000 cells per well in a 96-well plate. The testing facilities were as follows: humidified atmosphere with 5% CO₂ at 37°C. Incubation time equaled 24 hours. For the negative control, purified water without any components of the composition was used. The number of survived cells was registered with MTT at 570 nm. The number of biological replicates was 3.

At the second stage, the definition of the amount of Type 3 aquaporins in keratinocytes after cultivation under the standard conditions, using the maximal non-toxic concentration of the component from the previous text. To determine the amount of AQP3, a commercially available test with the use of sandwich-type enzyme-linked immunoassay was used. Cells of HaCaT line with the density of 10⁵ cells per well were introduced to the 96-well plate. On the next day, the cell medium was removed and replaced with DMEM + 5% FBS 50µL fresh cell medium to maintain cell growth. Next, 50µL of samples of the composition under the invention was added, and cultivation was performed for 24 hours. In all the tests, 0.20% sodium chloride was added to the cell medium to regulate osmotic balance and to create physiological conditions to cultivate skin cells. The cultivating environment conditions were as follows: humidified atmosphere with 5% CO₂ at 37°C. After incubation, cell supernatant was collected, and the amount of Type 3 aquaporins in skin keratinocytes was determined with ELISA method. Biotin-conjugated specific antibodies were bound with AQP3, next horse-radish peroxidases, conjugated to avidin was added in order to bind with antibodies and change color with TMB. Only after binding the 3 components of the reagent with AQP3 a stable color could be achieved, which was fixed with sulphuric acid and spectropotometrically detected at 450±10 nm wave length. For the negative control, purified water was used; for the positive control, Hydagen Aquaporin BASF, a mixture of glycerin and glyceryl glycoside, was used, which is known for its capacity to impact the number of aquaporins in skin cells (under the research made by BASF). Statistical data processing was used to calculate the average, the standard deviation and trends for every biological replication. Data normality was controlled with Shapiro-Wilk test. The controls were compared with non-parametric statistics, in particular, Wilcoxon test method.

### Results.

Following the primary cytotoxicity estimation results it was found out that the components of the composition, individually and in combination, do not cause the death of skin keratinocytes in the claimed concentration interval. The vitality of skin keratinocytes was preserved for the whole concentration range of the composition's components and above the threshold value 70%, which proves gentle impact on skin keratinocytes (Table No. 6). Joining *Aloe Barbadensis* leaf extract and trimethylglycine in 1: 1 mass ratio for the raw materials allowed increasing survivability of skin keratinocytes within the range of 0.03-10.0 weight % if compared with *Aloe Barbadensis* leaf extract and trimethylglycine used separately; this may prove strengthening of keratinocyte membrane and more favorable effect of the components on the metabolic activity of skin cells.

**Table No. 6. Cytotoxicity estimation for the components of the composition on skin cells**

| **Sample tested** | **Components of the composition** | **Vitality of cells, % when adding the components of the composition into the cultural medium (weight %)** | | | | |
|---|---|---|---|---|---|---|
| | | **0.03%** | **0.10%** | **0.25%** | **1.00%** | **10.00%** |
| No. 1 | Negative control (purified water) | 100.0+0.00 | 100.0+0.00 | 100.0+0.00 | 100.0+0.00 | 100.0+0.00 |
| No. 2 | Aloe Barbadensis leaf extract | 83.2+2.25 | 82.7+3.04 | 78.5+6.15 | 80.0+0.66 | 72.0+4.03 |
| No. 3 | Trimethylglycine | 98.8+3.47 | 99.9+1.47 | 95.4+2.94 | 93.5+1.66 | 90.3+6.82 |
| No. 4 | *Aloe Barbadensis* leaf extract + trimethylglycine 1:1 mass ratio for the raw material | 95.4±5.81 | 99.3±4.15 | 99.1±6.50 | 99.3±16.80 | 86.9±7.81 |

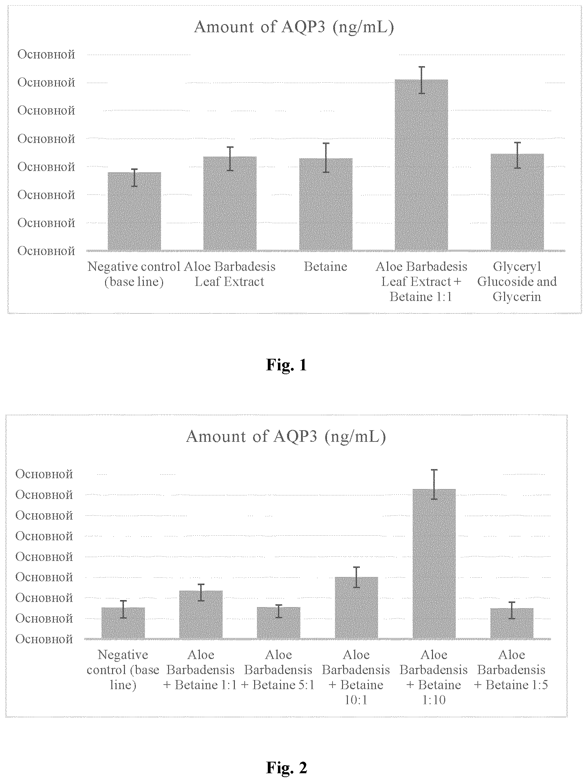

For further study, concentrations of components with largest survivability of skin cells were selected. Following the results of estimation of the amount of Type 3 aquaporins in skin keratinocytes after adding the components of the composition into the cultural medium, synergetic effect *of Aloe Barbadensis* leaf extract and trimethylglycine in 1:1 mass ratio for the raw material ratio equivalent to active ingredients is (0.0001-1.00):(0.1-10.0) weight %, if used jointly, was detected (see Fig. 1 - Definition of the amount of AQP3 in skin keratinocytes). A combination of *Aloe Barbadensis* leaf extract and trimethylglycine demonstrated synergetic effect and increased the amount of Type 3 aquaporins by +118.82% if compared to the control (Table No. 7), which proves long-term improvement of transepidermal water flow in skin cells.

**Table No. 7. Estimation of the amount of AQP3 in skin keratinocytes**

| **Sample tested** | **Components of the composition** | **Amount of AQP3 (ng/mL)** | **Increase of AQP3 amount in relation to the negative control, %** |
|---|---|---|---|
| No. 1 | Negative control (purified water) | 5.58±0.24 | - |
| No. 2 | *Aloe Barbadensis* leaf extract, 0,01% equivalent to the active ingredient | 6.73±0.69* | +20.61%* |
| No. 3 | Trimethylglycine, 1% equivalent to the active ingredient | 6.58±1.08 | +17.92% |
| No. 4 | *Aloe Barbadensis* leaf extract + trimethylglycine in 1:1 mass ratio for the raw material, 0.01%+1% was equivalent to the active ingredients | 12.21±0.91*** | +118.82%*** |
| No. 5 | Positive control (Hydagen Aquaporin, glycerin and glyceryl glucoside), 1% for the raw material | 6.89±0.82* | +23.48%* |
| Significance levels: *p<0.05; **p<0.01; ***p<0.001 | | | |

Additionally, combinations *of Aloe Barbadensis* leaf extract and trimethylglycine were studied in various mass ratios in joint use to check the hypothesis and to select the best component ratio in the formula. A concentration of trimethylglycine was fixed, and the ratio of *Aloe Barbadensis* leaf extract was multiplied to received mass ratios 1:1, 1:5, 1:10 for the raw materials. The authors unexpectedly discovered, that stimulation of AQP3 in epidermal cells is specified by quantitative ratio *of Aloe Barbadensis* leaf extract and trimethylglycine parts in their dependencies (Table No. 8). Thus, combinations *of Aloe Barbadensis* leaf extract and trimethylglycine in the ratios of 10:1 and 1:10 for the raw material demonstrated synergetic effect and increase the amount of Type 3 aquaporins by +98.35% and +380.20% if compared to the control (p<0.01), which proves the significant and long-term improvement of transepidermal water flow in skin cells and expressed increase of the aquaporin number for transepidermal water delivery (see Fig. 2 - Definition of the amount of AQP3 in skin keratinocytes for various ratios). It is interesting that the combination *of Aloe Barbadensis* leaf extract and trimethylglycine in 5:1 ratio for the raw material did not increase the amount of Type 3 aquaporins and remained them at the previous level in skin cells, which confirms the importance of the correct selection of component ratio in the formylation in order to receive significant skin moisturizing effect.

**Table No. 8. Estimation of the amount of AQP3 in skin keratinocytes in various component ratios**

| **Sample tested** | **Components of the composition** | **Amount of AQP3 (ng/mL)** | **Increase of the amount of AQP3 referring to the negative control, %** |
|---|---|---|---|
| No. 1 | Negative control (purified water) - basic aquaporin level in skin | 3.03±0.66 | - |
| No. 2 | *Aloe Barbadensis* leaf extract and trimethylglycine, 1:1 ratio for the raw | 4.69±0.60* | +54.79%* |
| | material, 0.01%+1% equivalent to the active ingredients | | |
| No. 3 | *Aloe Barbadensis* leaf extract and trimethylglycine, 5:1 ratio for the raw material, 0.05%+1% equivalent to the active ingredients | 3.06±0.25 - | +0.99% |
| No. 4 | *Aloe Barbadensis* leaf extract and trimethylglycine, 10:1 ratio for the raw material, 0.10%+1% equivalent to the active ingredients | 6.01±0.96** | +98.35%** |
| No. 5 | *Aloe Barbadensis* leaf extract and trimethylglycine, 1:10 ratio for the raw material, 0.01%+10% equivalent to the active ingredients | 14.55±1.88** - | +380.20%** |
| No. 6 | *Aloe Barbadensis* leaf extract and trimethylglycine, 1:5 ratio for the raw material, 0.01%+5% equivalent to the active ingredients | 2.97+0.60 | -1.98% |
| Significance levels: *p<0.05; **p<0.01 | | | |

Type 3 aquaporins (AQP3) in the basal and superbasal layer of epidermis provide regulation of transepidermal water flow by its own stimulation of transporting low molecular substances, in particular, water, to cells. A principally new yet natural skin cell moisturizing mechanism provides deep penetration of water flow in all epidermal layers and in the first dermis layer. Type 3 aquaporins are very sensitive to pH parameter of the product, heavy metals, UVA- and UVB-radiation, epigenetic factors; they are critical in development of dermatological disorders, in particular, atopic dermatitis, eczema, psoriasis, vitiligo.

The natural biocomplex based on *Aloe Barbadensis* leaf extract and trimethylglycine provides stimulation of AQP3 synthesis in epidermal keratinocytes and integration of aquaporins into cell membranes for water flow transportation. Increase of water content in skin cells stimulates metabolism, renewal and normal growth of keratinocytes in cells, and also prevents dehydration and excessive peeling of the skin, caused by lack of water in keratinocytes. The components help reducing transepidermal water loss, increasing the amount of Type 3 aquaporins in skin cells for long term prevention of dry skin, caused by specific impact of various environmental factors and the use of unsuitable cosmetic ingredients in the product composition. pH-sensitive regulation of Type 3 aquaporins allows preserving pH-gradient, Marcionini's mantle and buffer capacity of skin, decrease the activity of the corneal layer protease and preserve the barrier layer of the skin, as well as to keep hydrolipidic layer made of sebum on the skin surface.

The complex, in mass ratio 1:1 (1 weight % and 1 weight %), has a synergetic effect and stimulates the production of AQP3 in keratinocytes by +118,82% compared to the control, as well as compared with the well-known combination of glycerin and glyceryl glycoside, applied in this art. Change of the ratio *of Aloe Barbadensis* leaf extract and trimethylglycine up to 10:1 provides stimulation of AQP3 synthesis in epidermal keratinocytes by +380.20% (p<0.01). Joining the components of the composition allows impacting all the epidermal layers [Bollag W.B., Aitkens L., White J. et al. Aquaporin-3 in the Epidermis: More than Skin Deep // American Journal of Physiology-Cell Physiology, 2020], providing deep transepidermal water flow in all epidermal cells and allowing skin dehydration, in comparison with the equivalent products. Deep moisturizing means increase of transepidermal water flow in all 5 epidermal layers, where AQP3 are located. The synergetic effect is manifested in additional long-term regulation of pH-gradient of the skin acid mantle, decrease of TEWL and protection from negative environmental factors. *Aloe Barbadensis* leaf extract stimulates synthesis and integration of AQP3 to epidermal cell membranes, protects them from negative environmental factors, while trimethylglycine provides water conducting properties of the skin cells themselves, stabilizes native structure of AQP3 in cell membranes and make the epidermal corneal layer elastic. The complex does not interfere with cellular processes and does not damage cellular structure, impacts gene deep moisturizing processes in the skin itself and does not make an occlusive film on the cell surface, which is proved by the positive results of cytotoxicity study of skin keratinocytes.

The use of the complex in the composition of various cosmetic products for scalp and hair care allows reaching deep transepidermal water flow in all epidermal layers till the dermis layer, provide adaptivity mechanism for the natural moisturized condition, preserve the skin barrier layer and restore the skin barrier layer without surface occlusion.

### Example 7.

A clinical study was performed to estimate the efficiency of the composition for the invention, designed to restore the skin barrier function, decrease TEWL and epidermis moisture. A liquid hair and body wash, consisting of the components as per Table No. 2, was used as the basis to introduce the components.

The hair and body wash are a liquid product, used as a cleansing product for body, scalp and hair during hygienic procedures (taking a shower or a bath). The main cleansing components of such products are surfactants, the action of which is based on dissolution of skin contamination. With this, negative impact on skin condition can also occur, which can result in lack of epidermal moisture, irritation, dry skin, increased sensitivity up to skin dehydration. To decrease the negative impact of surfactants, a composition based on *Aloe Barbadensis* leaf extract and trimethylglycine is additionally introduced to the hair and body wash in order to improve the product's value for consumers.

To determine the claimed effects of the hair and body wash, containing the complex of *Aloe Barbadensis* leaf extract and N-trialkylated glycine derivative, in particular, trimethylglycine, a prospective, open-label, non-randomized, uncontrolled study was performed with participation of 15 subjects aged 20 - 62, the average age was 36±11.1 years old. To estimate the efficiency, the subject group used the hair and body wash under the individual hygienic routine. The subjects corresponded to the inclusion criteria and did not have any exclusion criteria. The study was performed in accordance with the Declaration of Helsinki and after signing the corresponding written consent.

Before the study, sensibilizing and irritating properties of the hair and body wash were estimated by a single application of the tested product with the composition for the invention skin under the control of a dermatologist. In case of a positive result, the subjects used the product with the composition at home for at least 7 days. The average period of the study for a subject was 12.5 days. At the end of this stage subject visited the study center to perform the estimation of the product with the composition after use and to answer the questionnaire on the product properties.

Before the study, subjects became acclimatized in the testing environment for 20-30 minutes at the temperature of (+10-+30°C)±1°C and air humidity of (5-85%)±2%. The moisturizing level and pH of the skin of the body were estimated on the external surface of the right shoulder, and the moisturizing level and pH of the scalp - in the area above the right auricle, visually with less amount of hair for a more accurate result. The effects were estimated with ASW 300 device (Aramo Smart Wizard, Medicinal Device Registration Certificate No. P3H2018/6812 dated January 10, 2020), designed for visual skin diagnostics (dermatoscopy).

Moisture content in all epidermal layers was quantitatively determined (%) in the conditions of electric current passage. Electrical conductivity and electric power between probe sensors, directly touching the skin surface, are transformed into digital data for measurement. The higher is moisture content in keratinocytes, the higher is conductance coefficient. Measurement was made with a handpiece with integrated moisture sensor with further analysis. Moisture meter was installed on skin until the sound signal. Next, software was used to analyze the acquired results.

To measure pH of the body skin and of the sculp, a portable acid meter was used (HI83141 pH/_{M}B/C-meter with HI1413B combined pH-electrode) to measure on the surface (glass body). The sensor was applied to the tested skin area, the results were shown in the device screen, introduced to the primary documentation and analyzed.

Additionally, consumer preference test of the product was performed with the use of a diagnostic questionnaire, demonstrating the consumer properties of the hair and body wash in routine use. The method suggested repetitive individual interviews with the subjects. The subjects were offered to ask the questions of the specially developed questionnaire. The questionnaire included questions about the consumer properties of the wash, as well as other questions, designed to detect special features of using the wash, to receive opinions and requests from the subjects.

For all the quantitative data, group mean value (M), median value (Me) and standard deviation (SD) were calculated. The variables presented as subjects' shares, were estimated in descriptive forms in percentages. The data on the parameters were presented based on the nature of the variable, continuous or categorial. The data of the categorial variables will be analyzed as the number (n) and the shares (percentage) of the total number of subjects, having a value of the analyzed parameter. The acquired results were processed with Statistica software package (StatSoft, USA, Ver. 8.0). For the statistical comparative criterion, Wilcoxon rank sum test was selected, which is used for non-parametric comparisons inside groups. The differences were considered statistically significant at the significance value p<0.05.

### Results.

Following the results of the epidermal layer moisture and pH of the body skin and scalp, it was found out that the tested composition in the formulation of the hair and body wash has an expressed effect of increasing epidermal layer moisture and pH regulation to provide pH-dependent stimulation of transepidermal water flow through Type 3 aquaporins, which is manifested in immediate increase of epidermis moisture by +236% in 5 minutes after using the product with the composition (Table No. 9). In the result of dermatoscopy, an immediate body skin and scalp moisturizing was observed in 93% of the subjects, which proves high clinical efficiency of the product with the composition for the invention. At the end of the study, 86% of the subjects observed statistically significant improvement of epidermal moisture by 42% (body skin) and 75% (scalp), or preservation of the effect without any changes (p<0.017), which proves long-term changes in the regulation of transepidermal water flow in all the epidermal layers. Considering the fact that at the baseline the subjects showed good epidermal moisture, the observed effect will be more remarkable in case of more dry or dehydrated skin, and the moisture index of the epidermal layer of the body skin and scalp will be significantly higher.

**Table No. 9. Estimation of the epidermal layer moisture within the clinical study**

| **Area** | **Components of the composition for the invention** | **Quantitative skin moisture index by measuring conductance coefficient of keratinocytes, %** | | | | |
|---|---|---|---|---|---|---|
| | | **Visit 1 Before use** | **Visit 1 After use (5 minutes)** | | **Visit 2 (10-14 days)** | |
| Body skin | 0.5% trimethylglycine + 0.0001%*Aloe Barbadensis* leaf extract | 9.67±4.98 | 32.53±14.88* | +236%** | 13.71±10.41 | +42% |
| Scalp | 0.5% trimethylglycine + 0.0001%*Aloe Barbadensis* leaf extract | 9.67±4.98 | 17.80±11.87* | +84%** | 16.93±11.08* | +75% |
| ^{∗}p<0.017 (considering the approach to the calculation: 0.05 divided into 3 visits); **p<0.005 | | | | | | |

Following the results of epidermal layer pH estimation, it was found out, that the hair and body wash with the components of the composition for the invention provides long-term pH regulation and shift of the parameter to a more acid area (Table No. 10). After the long-term use of the hair and body wash, the decrease of pH of the body skin was observed from 5.68±0.92 to 5.41±0.59, which proves pH-dependent regulation of body epidermis moisture and transepidermal water flow in skin cells. The decrease of pH of the body skin favors the differentiation of keratinocytes and epidermis renewal, support of the normal microflora of the skin, maintenance of hydrolipid mantle and prevention of atopic conditions of highly sensitive skin [Braun-Falco O., Korting H.C. Der normale pH-Wert der menschlichen // Haut. Hautarzt. - 1986. - Vol. 37. -P.126-129]. The decrease of pH of the scalp also favors the scalp condition, the decreased of TEWL, protection of hair from mechanical damages, maintenance of epidermis, seboregulation, preservation of biological diversity of the scalp microflora in order to prevent dandruff, seborrheic dermatitis and psoriasis [Braun-Falco O., Korting H.C. Der normale pH-Wert der menschlichen // Haut. Hautarzt. - 1986. - Vol. 37. - P. 126-129]. After long-term use of the hair and body wash with the components for the invention, decrease of the pH parameter of the scalp was detected from 5.68±0.92 to 5.25±0.40, as well as increase of epidermis moisture, which proves pH-dependent regulation of moisture of the body epidermis and transepidermal water flow in skin cells through Type 3 aquaporins. At the end of the study, in more than 57% and 64% of the subjects, statistically significant decrease of the body and scalp pH was observed (p<0.017), correspondingly; with this, no negative dermatological manifestations were absent, the body skin and the scalp preserved the hydrolipid barrier without suppressing the production of skin sebum.

**Table No. 10. Estimation of the epidermal layer pH within the clinical study**

| **Area** | **Components of the composition for the invention** | **Quantitative pH index, CUs** | | | | |
|---|---|---|---|---|---|---|
| | | **Visit 1 Before use** | **Visit 1 After use (5 minutes)** | | **Visit 2 (10-14 days)** | |
| Body skin | 0.5% trimethylglycine + 0.0001%*Aloe Barbadensis* leaf extract | 5.68±0.92 | 5.64±0.62 | -0.7% | 5.41±0.59 | -4.7% |
| Scalp | 0.5% trimethylglycine + 0.0001%*Aloe Barbadensis* leaf extract | 5.68±0.92 | 5.58±0.78 | -1.8% | 5.25±0.40 | -7.6% |

Following the results of the study, the composition based on *Aloe Barbadensis* leaf extract and N-trialkylated glycine derivative, in particular, trimethylglycine, allowed reaching high clinical efficiency through moisture increase in all the epidermal layers of the body skin and the scalp, as well as to provide immediate increase in moisture after the use of the product with the composition and regulate pH-balance of the body skin and scalp in order to increase transepidermal water flow in keratinocytes through AQP3. Joining the components in specific mass ratios allowed increasing the level of the skin moisture without lessening the oil gland activity and the production of the skin natural sebum. 87% of the subjects observed no skin tightness and dry skin after a single and a long-term use of the hair and body wash with the composition for the invention; and more than 87% subjects estimated the condition of the skin moisturizing after the product use as 7.33 out of 10, subjectively.

Following the results of the study, the composition based on *Aloe Barbadensis* leaf extract and trimethylglycine increased the epidermis moisture after a single and a long-term use of the product at the statistically significant level, which proves the reliable increase of skin moisture and positive impact of the product with the composition on the body skin and the scalp.

### Example 8.

A clinical study was performed to estimate the efficiency of the composition for the invention to restore the skin barrier function, decrease of TEWL and pH-dependent of epidermis moisture. A liquid body wash, comprising of the components as per Table No. 1, was used as the basis to introduce the components.

A shower gel is a liquid product, used as a cleansing product for body during hygienic procedures (taking a shower or a bath). The main cleansing components of such products are surfactants, the action of which is based on dissolution of skin contamination. With this, negative impact on skin condition can also occur, which can result in lack of epidermal moisture, irritation, dry skin, increased sensitivity up to skin dehydration. To decrease the negative impact of surfactants, a composition based on *Aloe Barbadensis* leaf extract and trimethylglycine is additionally introduced to the hair and body wash in order to improve the product's value for consumers.

To determine the claimed effects of the shower gel, containing the complex of *Aloe Barbadensis* leaf extract and N-trialkylated glycine derivative, in particular, trimethylglycine, a prospective, open-label, non-randomized study was performed with participation of 15 subjects aged 18-50. To estimate the efficiency, the subject group used the shower gel twice a day (in the morning and in the evening) for 30 days. The subjects corresponded to the inclusion criteria and did not have any exclusion criteria. The study was performed in accordance with the Declaration of Helsinki and after signing the corresponding written consent.

Before the study, subjects became acclimatized in the testing environment for 20-30 minutes at the temperature of (+22°C±4)°C and standard air humidity. The moisture level and pH of the skin of the body were estimated on the external surface of the forearm.

Differential diagnostics of transepidermal water flow was performed. The TEWL parameter of the epidermis was determined quantitively (%) with a tewameter. A tewameter allows determining the condition of skin barrier function and, correspondingly, transepidermal water loss. Normally, skin evaporates some quantity of water, which is the result of the metabolic processes occurring in skin. However, in case of the slightest damage to the skin barrier function, which is not visible by a human eye, water loss will increase. Additionally, the condition of the epidermis moisture was determined with a corneometric method. Next, a software was used to analyze the acquired results.

To measure pH of the body skin and of the sculp, a portable acid meter was used (HI83141 pH/_{M}B/C-meter with HI1413B combined pH-electrode) to measure on the surface (glass body). The sensor was applied to the tested skin area, the results were shown in the device screen, introduced to the primary documentation and analyzed.

For all the quantitative data, group mean value (M), median value (Me) and standard deviation (SD) were calculated. The acquired results were processed with Statistica software package (StatSoft, USA, Ver. 8.0). For the statistical comparative criterion, Wilcoxon rank sum test was selected, which is used for non-parametric comparisons inside groups. The differences were considered statistically significant at the significance value p<0.05.

### Results.

Following the results of the epidermal layer moisture and pH of the body skin and scalp, it was found out that the tested composition in the formulation of the hair and body wash has an expressed effect of increasing epidermal layer moisture and pH regulation to provide pH-dependent stimulation of transepidermal water flow through Type 3 aquaporins, which is manifested in immediate increase of epidermis moisture by +7.04% in 30 days after using the product with the composition (Table No. 11). According to a dermatologist's statement, the shower gel with the composition for the invention does not cause excessive water loss, does not over dry skin and does not cause skin tightness. The subjects observed no dry skin, no tightness and discomfort after use. At the end of the study, statistically significant improvement of epidermis moisture was observed, which proves long-term changes in regulation of transepidermal water flow in all the epidermal layers. Considering the fact that at the baseline the subjects showed good epidermal moisture, and the time of contact of the washable product made max. 3 minutes in the morning and in the evening, the observed effect will be more remarkable in case of more dry or dehydrated skin, and the moisture index of the epidermal layer of the body skin and scalp will be significantly higher.

**Table No. 11. Estimation of the epidermal layer moisture within the clinical study**

| **Area** | **Components of the composition for the invention** | **Quantitative skin moisture index by measuring conductance coefficient of keratinocytes, %** | | |
|---|---|---|---|---|
| | | **Visit 1 Before use** | **Visit 2 After 30 days of use** | **Change** |
| Body skin | 0.5% trimethylglycine + 0.0001%*Aloe Barbadensis* leaf extract | 22.5 | 22.8 | +1.33% |
| | 1% trimethylglycine + 0.0001%*Aloe Barbadensis* leaf extract | 20.1 | 21.2 | +5.47% |
| | 2% trimethylglycine + 0.0001%*Aloe Barbadensis* leaf extract | 21.3 | 22.8 | +7.04% |

Following the estimation of the skin barrier function and the TEWL it was found out that the tested composition in the liquid shower gel has an expressive effect of decreasing transepidermal water loss and restoring the skin barrier function, which is manifested in decrease of TEWL by 6,40% after 30 days of the use of the product with the composition (Table No. 12). According to a dermatologist's statement, the shower gel with the composition for the invention does not cause excessive water loss, does not over dry skin and does not cause skin tightness. The subjects observed no dry skin, no tightness and discomfort after use. At the end of the study, statistically significant improvement of epidermis moisture was observed, which proves long-term changes in regulation of transepidermal water flow in all the epidermal layers and restoration of the skin barrier. Considering the fact that at the baseline the subjects showed good epidermal moisture, and the time of contact of the washable product made max. 3 minutes in the morning and in the evening, the observed effect will be more remarkable in case of more dry or dehydrated skin, and the moisture index of the epidermal layer of the body skin and scalp will be significantly higher.

**Table No. 12. Estimation of TEWL within the clinical study**

| **Area** | **Components of the composition for the invention** | **Quantitative TEWL index by measuring the amount of evaporated water with a tewameter (evaporimeter), g/m2h** | | |
|---|---|---|---|---|
| | | **Visit 1 Before use** | **Visit 2 After 30 days of use** | **Change** |
| Body skin | 1% trimethylglycine + 0.0001%*Aloe Barbadensis* leaf extract | 4.42 | 4.31 | -2.49% |
| | 2% trimethylglycine + 0.0001%*Aloe Barbadensis* leaf extract | 12.5 | 11.7 | -6.40% |

Following the results of estimation of pH of the epidermal layer, it was found out that the shower gel with the components of the composition for the invention provides long-term pH regulation and shift of the parameter to a more acid area (Table No. 13). After the long-term use of the hair and body wash, the decrease of pH of the body skin was observed from to 5.6%, which proves pH-dependent regulation of body epidermis moisture and transepidermal water flow in skin cells, as well as pH-regulation for normal microflora. The decrease of pH of the body skin favors the differentiation of keratinocytes and epidermis renewal, support of the normal microflora of the skin, maintenance of hydrolipid mantle and prevention of atopic conditions of highly sensitive skin [Braun-Falco O., Korting H.C. Der normale pH-Wert der menschlichen // Haut. Hautarzt. - 1986. - Vol. 37. - P. 126-129]. At the end of the study, a dermatologist noted the decrease of the body skin pH with the absence of negative dermatological events; the body skin preserved its hydrolipid barrier without any skin discomfort after use.

**Table No. 13. Estimation of the epidermal layer pH within the clinical study**

| **Area** | **Components of the composition for the invention** | **Quantitative pH index, CUs** | | |
|---|---|---|---|---|
| | | **Visit 1 Before use** | **Visit 2 After 30 days of use** | **Change** |
| Body skin | 0.5% trimethylglycine + 0.0001%*Aloe Barbadensis* leaf extract | 5.64 | 5.40 | -4.26% |
| | 1% trimethylglycine + 0.0001%*Aloe Barbadensis* leaf extract | 5.96 | 5.68 | -4.70% |
| | 2% trimethylglycine + 0.0001%*Aloe Barbadensis* leaf extract | 5.36 | 5.06 | -5.60% |

Following the results of the study, the composition based on *Aloe Barbadensis* leaf extract and N-trialkylated glycine derivative, in particular, trimethylglycine, allowed reaching high clinical efficiency through moisture increase in all the epidermal layers of the body skin and the scalp, as well as to provide TEWL decrease and regulate pH-balance of the body skin and scalp in order to increase transepidermal water flow in keratinocytes through AQP3. Joining the components in specific mass ratios allowed increasing the level of the skin moisture. Subjects gave a positive estimation of the consumer properties of the product.

Following the results of the study, the composition based on *Aloe Barbadensis* leaf extract and trimethylglycine increased the epidermis moisture after a long-term use of the product, decreased TEWL and regulated pH, which proves the reliable increase of skin moisture and positive impact of the product with the composition on the body skin.

### Example 9.

A clinical study was performed to estimate the efficiency of the composition for the invention to restore the barrier function of the hair, in particular, the scalp, epidermis moisture, hair moisture and porosity (its ability to absorb nutrients). A hair shampoo and a hair balm, consisting of the components as per Tables No. 4 and No. 5, were used as the basis to introduce the components.

A shampoo and a balm are liquid products, used as hair cleansing and hare care product for hair and sculp during hygienic procedures. The main cleansing components of a shampoo are surfactants, the action of which is based on dissolution of sculp and sebum regulation. With this, negative impact on the hair can also occur, which can result in lack of epidermal moisture, irritation, dry skin (xerosis), increased sensitivity and occurrence of dandruff. To decrease the negative impact of surfactants, the formulation has additional composition based on *Aloe Barbadensis* leaf extract and trimethylglycine in order to improve the product's value for consumers. Moreover, the components of the shampoo can increase the hair porosity, thus decreasing water retaining capacity and capacity to absorb active ingredients.

To determine the claimed effects of the shampoo and the balm, containing the complex of *Aloe Barbadensis* leaf extract and N-trialkylated glycine derivative, in particular, trimethylglycine, a prospective, open-label, non-randomized study was performed with participation of 15 subjects. To estimate the efficiency, the subject group used the shampoo and the balm together at least 2 times a week during 60 days. The subjects corresponded to the inclusion criteria and did not have any exclusion criteria. The study was performed in accordance with the Declaration of Helsinki and after signing the corresponding written consent.

Before the study, subjects became acclimatized in the testing environment for 20-30 minutes at the temperature of (+22°C±4)°C and standard air humidity. The moisture level of the scalp was performed in the area above the right auricle, visually with less amount of hair for a more accurate result.

Differential diagnostic of transepidermal water flow was performed with a corneometric method. Corneometrics is rather a simple method, allowing fast and effective determination of the skin reaction to water retaining action, but additional studies are necessary to use this method when selecting a treatment. Next, a software was used to analyze the acquired results.

To measure hair porosity as capacity to absorb water and nutrients and preserve hair volume without using additional styling products, a water test and a tactile test were used. The result was estimated in accordance with the recommendation on hair porosity estimation (Table 14).

**Table No. 14. Hair porosity estimation: interpretation of the results**

| **Porosity** | **Hair hydration** | **Cuticle condition** | **Moisture and ability to absorb nutrients (physiological characteristics)** | **Ability to preserve hair volume (aesthetic characteristics)** |
|---|---|---|---|---|
| Low | Hydrophobic | Severely closed | Small degree of nutrient and water absorption | Cannot preserve hair volume |
| Normal | Optimal hydration | Non-damaged cuticle (healthy hair) | Moderately moisturized; sufficient degree of water and nutrient absorption and retaining | Average period of hair volume preservation |
| High | Hydrophilic | Damaged and open | Absorbs water easily, but does not retain it and becomes dry | Good preservation of hair volume |

For all the quantitative data, group mean value (M) was calculated and compared with the baseline values according to the measurement results. The acquired results were processed with Statistica software package (StatSoft, USA, Ver. 8.0). For the statistical comparative criterion, Wilcoxon rank sum test was selected, which is used for non-parametric comparisons inside groups. The differences were considered statistically significant at the significance value p<0.05.

### Results.

Following the results of the epidermal layer moisture of the scalp and hair and the hair porosity, it was found out that the tested composition in the formulation of the hair shampoo and balm has an expressed effect of increasing epidermal layer moisture restoration of the barrier function of the hair and scalp, which is manifested in immediate increase of epidermis moisture by +27.7% in 60 days after joint use of the product with the composition (Table No. 15).

**Table No. 15. Estimation of the epidermal layer moisture within the clinical study**

| **Area** | **Components of the composition for the invention** | **Quantitative skin moisture index by measuring conductance coefficient of keratinocytes, %** | | |
|---|---|---|---|---|
| | | **Visit 1 Before use** | **Visit 2 After 60 days of use** | **Change** |
| Scalp | **Shampoo** | 18.0 | 23.0 | +27.7% |
| | 0.2% trimethylglycine + 0.005%*Aloe Barbadensis* leaf extract | | | |
| | **Balm** | | | |
| | 0.3% trimethylglycine + 0.005%*Aloe Barbadensis* leaf extract | | | |

According to a dermatologist's statement, the hair shampoo and balm with the composition for the invention does not cause irritative and sensibilization effect on the hair and the scalp. During the use, the improvement of moisture parameters of the hair and scalp, the hair porosity and hair moisture were observed. For more improvement and maintenance of the normal structure of hair it is recommended a longer cyclical use of the shampoo and balm with the composition for minimum 3 months.

Following the estimation of the hair porosity, it was found out that the tested composition in the formulation of the hair shampoo and balm with the composition has an expressive effect of decreasing hair porosity and increasing hair moisture (Table No. 16). At the end of the study, significant normalization of hair porosity to retain water in the hair and to better absorb active ingredients from the hair shampoo and balm were observed in all the subjects.

**Table No. 16. Estimation of hair porosity within the clinical study**

| **Area** | **Components of the composition for the invention** | **Hair porosity under Table No. 13** | | |
|---|---|---|---|---|
| | | **Visit 1 Before use** | **Visit 2 After 60 days of use** | **Change** |
| Hair | **Shampoo** | High Absorbs water easily, but does not retain it and becomes dry | Normal Normally moisturizes Sufficient degree of water and nutrient absorption and retaining | Normalization of hair porosity, increase of hair moisture and ability to absorb active ingredients |
| | 0.2% trimethylglycine + 0.005%*Aloe Barbadensis* leaf extract | | | |
| | **Balm** | | | |
| | 0.3% trimethylglycine + 0.005%*Aloe Barbadensis* leaf extract | | | |

Following the results of the study, the composition based on *Aloe Barbadensis* leaf extract and N-trialkylated glycine derivative, in particular, trimethylglycine, allowed reaching high clinical efficiency through moisture increase in the scalp and hair, normalization of hair porosity and moisture. Joining the components in specific mass ratios allowed increasing the level of the hair and scalp moisture. Subjects gave a positive estimation of the consumer properties of the product.

Following the results of the study, the composition based on *Aloe Barbadensis* leaf extract and trimethylglycine increased the epidermis moisture and water-retaining capacity of the hair after a long-term use of the product, which proves the positive impact of the product with the composition on the hair and scalp.

### Example 10.

A clinical study was performed to estimate the hypoallergenicity of the composition for the invention. A shower gel and a hair and body wash, consisting of the components as per Tables No. 1 and No. 2, were used as the basis to introduce the components.

To determine the hypoallergenicity and sensibilizing action of the shower gel and the hair and body wash, containing the complex *of Aloe Barbadensis* leaf extract and N-trialkylated glycine derivative, in particular, trimethylglycine, a prospective, open-label, non-randomized study was performed with participation of 15 subjects. To estimate the efficiency, the subject group used the shampoo and the balm together at least 2 times a week during 60 days. The subjects corresponded to the inclusion criteria and did not have any exclusion criteria. The study was performed in accordance with the Declaration of Helsinki and after signing the corresponding written consent.

Hypoallergenicity is a characteristic meaning that the content of substances, which could result in negative responses, is minimized. Manufacturers exclude ingredients, previously noted as irritant agents (allergenic agents) due to idiosyncrasy, from the formulation. Hypoallergenicity is a complex estimation confirming the absence of adverse response.

To determine the hypoallergenicity of the product, containing the complex of *Aloe Barbadensis* leaf extract and N-trialkylated glycine derivative, in particular, trimethylglycine, a prospective, open-label, non-randomized study was performed with participation of 15 subjects aged 18-50. The subjects corresponded to the inclusion criteria and did not have any exclusion criteria. The study was performed in accordance with the Declaration of Helsinki and after signing the corresponding written consent.

Before the study, subjects became acclimatized in the testing environment for 20-30 minutes at the temperature of (+22°C±4)°C and standard air humidity. The moisture level of the scalp was performed in the external area of the forearm.

Each subject was given a skin test by a drop method (the primary application). After 24 hours, next to the primary application (at a distance of at least 2 cm) on a "clean" area of the skin, in the absence of subjective symptoms or objective signs of irritation, a secondary application was placed to detect a sensitizing effect. Assessment of the functional state of the skin on the application spot was performed by visual inspection in 2, 24, 48 and 72 hours. Simultaneously with the tests, a control skin test with distilled water was performed. Next, to assess the "hypoallergenicity" of this product, a "provocative test" was continued daily, starting from the 4^{th} to the 12^{th} day to identify a sensitizing effect with potential allergens and a possible allergic reaction of a delayed type - contact allergic dermatitis. With active sensitization with small doses of the irritant, included into the product, the peak of the body sensitivity to it is reached by the 10^{th}-14^{th} day. The presence or absence of an erythematous reaction was visually recorded when examining the subject by a dermatologist.

### Results.

Following the result of body hypoallergenicity estimation, the absence of clinical manifestations of allergic reaction after a long-term use within 30 days and the conformance for people with sensitive and responsive skin was confirmed (Table No. 17). None of the subjects with sensitive skin had erythema, increased sensitivity, allergic reaction, which proves hypoallergenic effect of the products with the composition for the invention.

**Table No. 17. Estimation of hypoallergenicity of shower gels within the clinical study**

| **Area** | **Components of the composition for the invention** | **Estimation of irritative index, points** | | | | | |
|---|---|---|---|---|---|---|---|
| | | **In 2 hours** | **1 day** | **2 days** | **3 days** | **4 days** | **5 days** |
| Body skin | 0.5% trimethylglycine + 0.0001%*Aloe Barbadensis* leaf extract | 0 | 0 | 0 | 0 | 0 | 0 |
| | 1.0% trimethylglycine + 0.0001%*Aloe Barbadensis* leaf extract | 0 | 0 | 0 | 0 | 0 | 0 |
| | 2.0% trimethylglycine + 0.0001%*Aloe Barbadensis* leaf extract | 0 | 0 | 0 | 0 | 0 | 0 |

According to a dermatologist's statement, the shower gels and the hair and body washes with the composition for the invention does not cause irritative and sensibilization effect on the body skin. During the use, no dry skin, irritation or discomfort was observed.

Following the results of the study, the composition based on *Aloe Barbadensis* leaf extract and trimethylglycine had necessary claimed effects in the invention after a single and a multiple use of the product, having safe dermatological status for people with sensitive skin prone to allergic reactions.

## Claims

1. A composition comprising a complex of the following two active ingredients (A) and (B):
(A) Plant extract from Aloe Barbadensis, preferably an Aloe Barbadensis leaf extract, further preferably having the CAS number 85507-69-3 or 94349-62-9; and
(B) N-trialkyl glycine derivative, preferably trimethylglycine, further preferably having the CAS number 107-43-7,
wherein the raw material ratio equivalent to active ingredients of the components (A) and (B) as per wt.% is the following:
| (A) | : | (B) |
|---|---|---|
| (0.0001-1.00) | | (0.1-10) |
and wherein the (A):(B) raw material ratio is
- higher 1:5, preferably higher 1:6, further preferably higher 1:7; further preferably higher 1:8, further preferably higher 1:9, further preferably higher 1:10; or
- lower 1:5, preferably lower 1:6, further preferably lower 1:7; further preferably lower 1:8, further preferably lower 1:9, further preferably lower 1:10.

2. Pharmaceutical preparation comprising the composition of claim 1.

3. The pharmaceutical preparation of claim 2, wherein said pharmaceutical preparation is formulated for application on a skin of a subject.

4. The composition of claim 1, wherein said composition is for use as a medicament.

5. The composition for use of claim 4, wherein said composition is formulated for application on a skin of a subject.

6. The composition of claim 1 for use in the treatment of a subject suffering from a skin wound and/or suffering from at least one of the following skin diseases or symptoms:
- dermatitis, including seborrheic and atopic dermatitis;
- psoriasis;
- eczema;
- vitiligo;
- xerosis;
- excessive scaling of the skin, including dandruff of the scalp;
- proliferative hyperkeratosis;
- skin itching.

7. The composition for use of claim 6, wherein said composition is formulated for application on a skin of a subject.

8. The pharmaceutical preparation of claim 3 or the composition for use of any of claims 5-7, wherein said formulation is selected from the following:
- film;
- aerosol;
- suspension,
- solution,
- tincture,
- cream,
- paste,
- lotion,
- ointment,
- gel,
wherein the aforementioned formulations are preferably skin adhesive formulations.

9. The pharmaceutical preparation of claim 3 or the composition for use of any of claims 5-7, wherein said composition reduces the transepidermal moisture loss of the skin and/or increases the number of aquaporins type 3 (AQP 3) in all layers of the skin epidermis.

10. A body care product comprising the composition of claim 1.

11. The body care product of claim 10, wherein said body care product is selected from the group consisting of:
- cosmetic;
- perfume;
- gel, including shower gel;
- shampoo, including shampoo for scalp and hair care;
- balm;
- conditioner, including hair conditioner;
- soap, including liquid soap for hand washing;
- cleanser, including facial cleanser;
- cream, including cream for face, body and hand care;
- serum for face care;
- solid product for face, hand, and hair care.

12. The body care product of claim 10 or claim 11, wherein said body care product is for application to the body's skin and wherein the application of said body care product on the skin of the body reduces the transepidermal moisture loss of the skin and/or increases the number of aquaporins type 3 (AQP 3) in all layers of the skin epidermis.

13. Non-medical use of the composition of claim 1, wherein said use of said composition is
i) in cosmetology;
ii) as a component of a body care product, wherein said body care product is preferably selected from the group consisting of:
- cosmetic;
- perfume;
- gel, including shower gel;
- shampoo, including shampoo for scalp and hair care;
- balm;
- conditioner, including hair conditioner;
- soap, including liquid soap for hand washing;
- cleanser, including facial cleanser;
- cream, including cream for face, body and hand care;
- serum for face care;
- solid product for face, hand, and hair care;
iii) for body and head skin care;
iv) for distribution of transepidermal moisture flow in body and skin cells without disturbing the elastic-viscous-plastic properties of the epidermis cells.
v) regulating transepidermal water flow between cells throughout the epidermis, including promoting a decrease in transepidermal water loss;
vi) reducing transepidermal moisture loss (TEWL) in dehydrated skin.
